(19) Europäisches Patentamt
European Patent Office
Office européen des brevets

(11) Numéro de publication: **0 338 937 B1**

(12) # FASCICULE DE BREVET EUROPEEN

(45) Date de publication de fascicule du brevet: **08.12.93** (51) Int. Cl.$^5$: **C07D 295/08**, A61K 31/395

(21) Numéro de dépôt: **89401135.2**

(22) Date de dépôt: **21.04.89**

Le dossier contient des informations techniques présentées postérieurement au dépôt de la demande et ne figurant pas dans le présent fascicule.

(54) **Dérivés du [(hydroxy-4 phényl)-3 hydroxy-1 propyl-1]-1 benzène substitué en position 2 par une chaîne aminoalkylèneoxy, leur procédé de préparation et leur application en thérapeutique.**

(30) Priorité: **22.04.88 FR 8805382**

(43) Date de publication de la demande: **25.10.89 Bulletin 89/43**

(45) Mention de la délivrance du brevet: **08.12.93 Bulletin 93/49**

(84) Etats contractants désignés: **AT BE CH DE ES FR GB GR IT LI LU NL SE**

(56) Documents cités:
**FR-A- 2 518 992**
**FR-A- 2 537 970**

(73) Titulaire: **SYNTHELABO**
**22, Avenue Galilée**
**F-92350 Le Plessis Robinson(FR)**

(72) Inventeur: **Ward, Mona**
**Résidence Gambetta, 1-2, rue Henri Regnault**
**F-92400 Courbevoie(FR)**
Inventeur: **Settembre, Pierre-André**
**14, rue Jean Macé**
**F-78800 Houilles(FR)**
Inventeur: **Renaud, Alain**
**16, place des Arts**
**F-92500 Rueil Malmaison(FR)**
Inventeur: **Langlois, Michel**
**4, place César Franck**
**F-78350 Buc(FR)**

(74) Mandataire: **Kedinger, Jean-Paul et al**
**c/o Cabinet Malemont**
**42, avenue du Président Wilson**
**F-75116 Paris (FR)**

**EP 0 338 937 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouveaux dérivés du [(hydroxy-4 phényl)-3 hydroxy-1 propyl-1]-1 benzène substitué en position 2 par une chaîne aminoalkylèneoxy, leur procédé de préparation et leur application en thérapeutique.

Plus précisément, les dérivés selon l'invention répondent à la formule :

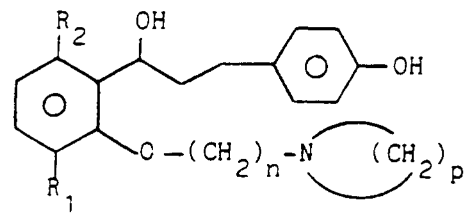

(I)

dans laquelle :

- $R_1$ et $R_2$ sont tels que :
  . soit $R_1$ représente un groupe méthoxy auquel cas $R_2$ est un groupe choisi parmi les suivants : hydroxyle : alkyle en $C_1$-$C_4$ ; alkyloxy en $C_2$-$C_8$ : cycloalkyloxy en $C_5$-$C_7$ ; benzyloxy ;
  . soit $R_2$ représente un groupe méthoxy auquel cas $R_1$ représente un groupe choisi parmi les suivants : hydroxyle ; alkyloxy en $C_2$-$C_8$ ; alkyloxy en $C_2$-$C_8$ substitué par un groupe méthoxy ; trifluoroalkyloxy en $C_1$-$C_4$ ; alcényloxy en $C_3$-$C_4$ ; cycloalkyloxy en $C_5$-$C_7$ ; benzyloxy ; alkylthio en $C_1$-$C_4$ ;
- n = 2 ou 3 ; et
- p = 4, 5 ou 6.

On connaît déjà des dérivés de ce type d'après FR-A-2 518 992 et FR-A-2 537 970. Ces documents toutefois n'exemplifient aucun des composés de formule (I) ci-dessus.

L'invention concerne également les sels d'addition d'acide de ces dérivés, ces sels pouvant être formés avec des acides minéraux pharmaceutiquement acceptables tels que l'acide chlorhydrique, sulfurique ou phosphorique ou avec des acides organiques pharmaceutiquement acceptables tels que l'acide fumarique, maléique, succinnique, oxalique, citrique ou tartrique.

Dans ce qui précède et ce qui suit, l'expression "alkyle en $C_1$-$C_4$" comprend notamment les groupes méthyle, éthyle, n-propyle et n-butyle ; l'expression "alkyloxy en $C_2$-$C_8$" comprend notamment les groupes éthoxy, n-propyloxy, n-butyloxy, n-pentyloxy, n-hexyloxy, n-heptyloxy et n-octyloxy ; l'expresson "cycloalkyloxy en $C_5$-$C_7$" comprend les groupes cyclopentyloxy, cyclohexyloxy et cycloheptyloxy ; l'expression "alcényloxy en $C_3$-$C_4$" comprend en particulier les groupes $OCH_2$-$CH=CH_2$, $OCH_2$-$CH=CH$-$CH_3$ et $OCH_2$-$CH_2$-$CH=CH_2$ ; et l'expression "alkylthio en $C_1$-$C_4$" comprend notamment les groupes méthylthio, éthylthio, n-propylthio et n-butylthio.

Entrent notamment dans le cadre de la présente invention, les composés et sels objet des revendications 2 et 3.

La présente invention a par ailleurs pour objet les procédés de synthèse des dérivés de formule (I), ces procédés étant conformes aux schémas réactionnels ci-après et aux revendications 6 à 15.

La présente invention s'étend en outre aux intermédiaires de synthèse de formules (II) et (III) apparaissant dans lesdits schémas réactionnels et notamment ceux faisant l'objet des revendications 16 et 17.

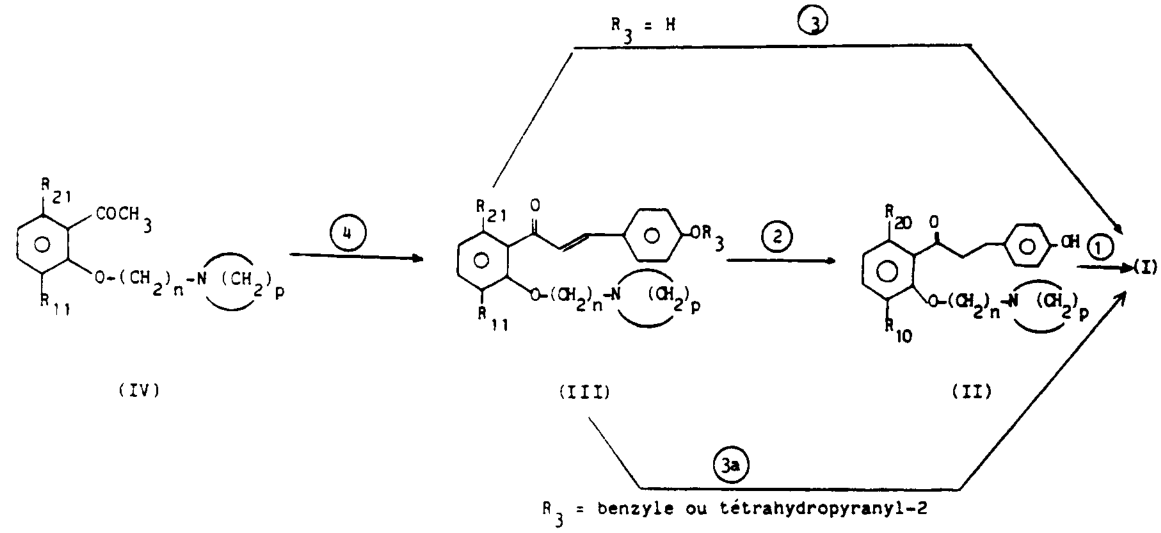


(IV) (III) (II)

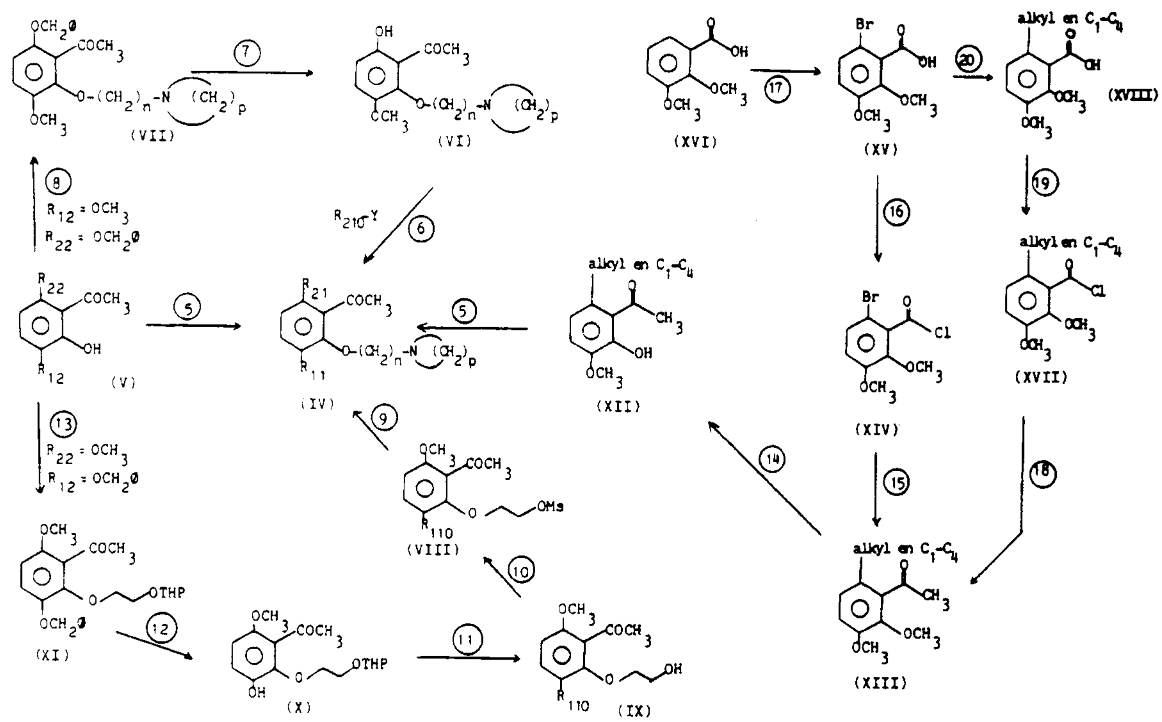


(VII) (VI) (XVI) (XV) (XVIII) (V) (IV) (XII) (XIV) (XVII) (VIII) (XI) (X) (IX) (XIII)

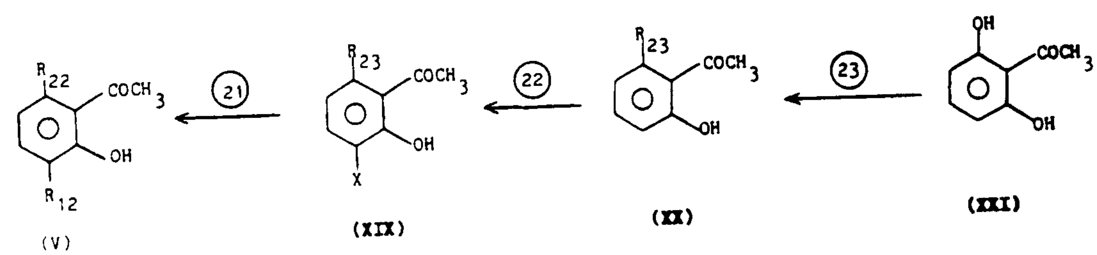

(V) (XIX) (XX) (XXI)

Dans les schémas réactionnels ci-dessus, les paramètres apparaissant dans les formules (II) à (XX) possèdent les significations suivantes :

- n = 2 ou 3,
- p = 4, 5 ou 6,
- $R_3$ = H, tétrahydropyranyl-2 ou benzyle,
- $R_{10}$ et $R_{20}$ ont respectivement la même signification que $R_1$ et $R_2$ dans la formule (I), à l'exception du groupe benzyloxy,
- $R_{11}$ et $R_{21}$ ont respectivement la même signification que $R_1$ et $R_2$ dans la formule (I), à l'exeption du groupe hydroxyle,
- $R_{210}$ = alkyle en $C_2$-$C_8$ ou cycloalkyle en $C_5$-$C_7$,
- Y = halogène, $OSO_2CH_3$ ou $OSO_3C_2H_5$,
- $R_{110}$ = alkyloxy en $C_2$-$C_8$ ou trifluoroalkyloxy en $C_1$-$C_4$,
- Ms = mésyle
- THP : tétrahydropyranyl-2,
- $R_{12}$ et $R_{22}$ sont tels que soit $R_{12}$ = $OCH_3$ auquel cas $R_{22}$ = benzyloxy, soit $R_{22}$ = $OCH_3$ auquel cas $R_{12}$ = alkyloxy en $C_2$-$C_8$, alkyloxy en $C_2$-$C_8$ substitué par un groupe méthoxy, alcényloxy en $C_3$ ou $C_4$, cycloalkyloxy en $C_5$-$C_7$, benzyloxy ou alkylthio en $C_1$-$C_4$,
- $R_{23}$ = $OCH_3$ ou benzyloxy,
- X = Br ou I.

Par ailleurs, les indices ① à ㉓ apparaissant dans ces schémas réactionnels, ont les significations suivantes :

① Réduction par le borohydrure de sodium, notamment en milieu alcoolique, de préférence éthanolique, en présence d'une base, par exemple NaOH,

② Hydrogénation, notamment dis l'éthanol, en présence de palladium acide sur charbon,

③ Réduction par le borohydrure de sodium dans l'éthanol en présence de pyridine et de NaOH,

(3a) Réduction par le borohydrure de sodium dis l'éthanol, suivie soit d' une hydrolyse acide lorsque $R_3$ = tétrahydropyranyl-2, soit d'une hydrogénolyse en présence de palladium sur charbon dans l'éthanol lorsque $R_3$ = benzyle,

④ Condensation de la benzaldéhyde de formule :

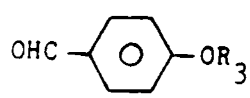

sur les composés (IV) en milieu alcalin dis l'éthanol ; lorsque $R_3$ = H, on utilise de préférence 5 équivalents de NaOH à 50 %, alors que lorsque $R_3$ = benzyle ou tétrahydropyranyl-2, 2,5 équivalents de NaOH à 40 % sont suffisants,

⑤ Condensation du composé de formule :

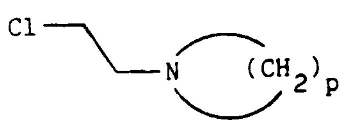

de préférence sous forme de chlorhydrate, sur les composés (V) pour lesquels $R_{22}$ = méthoxy et $R_{12}$ = alkyloxy en $C_2$-$C_8$, alkyloxy en $C_2$-$C_8$ substitué par un groupe méthoxy, alcènyloxy en $C_3$ ou $C_4$, cycloalkyloxy en $C_5$-$C_7$, benzyloxy ou alkylthio en $C_1$-$C_4$, de préférence dans un solvant organique aprotique tel que l'acétonitrile et en présence de carbonate de métal alcalin tel que le carbonate de potassium,

⑥ O-alcoylation de préférence dans un solvant organique tel que l'acétonitrile et en présence d'une base telle que le carbonate de potassium,

⑦ Débenzylation par action de l'hydrogéne en présence de palledium sur charbon, en milieu éthiolique,

⑧ Condensation du composé de formule :

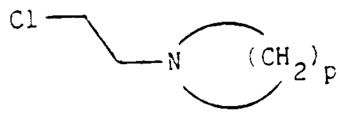

de préférence sous forme de chlorhydrate, sur les composés (V) pour lesquels $R_{12}$ = $OCH_3$ et $R_{22}$ = benzyloxy, dans les mêmes conditions opératoires qu'à l'étape ⑤,

⑨ Condensation avec l'amine de formule :

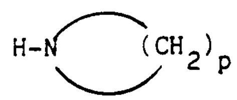

utilisée en excès et à reflux,

⑩ Action du chlorure de mésyle dans un solvant organique en présence de triéthylamine à froid,

⑪ Condensation sur les composés (X) du triflate de trifluoroalkyle en $C_1$-$C_4$ dans l'acétone en présence de $K_2CO_3$ ou d'un' halogénure d'alkyle en $C_2$-$C_8$ dans le DMF en présence de $K_2CO_3$, suivie d'une hydrolyse acide,

⑫ Action de l'hydrogéne en présence de palladium sur charbon dans l'éthanol,

⑬ Action du composé de formule :

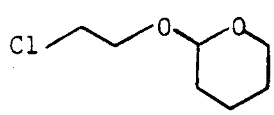

dans l'acétonitrile en présence de $K_2CO_3$ sur les composés (V) pour lesquels ($R_{12}$, $R_{22}$) = ($OCH_2Ø$, $OCH_3$),

⑭ Déméthylation par le chlorure d'aluminium, de préférence dans le dichlorométhane,

⑮ Condensation avec 2 équivalents de $CH_3MnI$ dans l'éther éthylique,

⑯ Action du chlorure de thionyle dans le toluène,

⑰ Action du brome, en présence de NaOH aqueuse, suivie d'une acidification par $H_2SO_4$ au reflux,

⑱ Condensation avec un équivalent de $CH_3MnI$ dans l'éther éthylique,

⑲ Action du chlorure de thionyle dans le toluène,

⑳ Alcoylation par un halogénure d'alkyle en $C_1$-$C_4$ en présence de deux équivalents de n-butyllithium,

㉑ Condensation avec les composés de formule :

$R_{120}$ - O - Na

ou

$R_{121}$ - S - Na

où $R_{120}$ = alkyle en $C_2$-$C_8$;alkyle en $C_2$-$C_8$ substitué par un groupe méthoxy ; alcènyle en $C_3$ ou $C_4$; cycloalkyle en $C_5$-$C_7$; ou benzyle, et

$R_{121}$ = alkyle en $C_1$-$C_4$,

en présence de CuI (quand $R_{120}ONa$ est mis en oeuvre) ou de $Cu_2O$ (quand $R_{121}SNa$ est mis en oeuvre),

㉒ Action du brome dans $CHCl_3$ ou du chlorure d'iode dans l'acide acétique,

㉓ O-alcoylation par action du chlorure ou bromore de benzyle ou du sulfate de méthyle, dans l'acétone en présence de $K_2CO_3$.

On précisera que les composés (I) pour lesquels $R_1$ = OH, peuvent encore être préparés par hydrogénolyse par le palladium sur charbon, en milieu éthanolique, des composés (I) correspondants pour

lesquels $R_1$ = O-$CH_2$Ø.

Les sels des dérivés de formule (I) sont obtenus de manière classique, par exemple par action d' un acide minéral ou organique en solution dans un solvant approprié sur lesdits dérivés eux-mêmes en solution dans un solvant spproprié.

Exemple 1 : Hydroxy-2 méthoxy-6 acétophénone [(XX) ; $R_{23}$ = $OCH_3$]
Numéro de code : 9

On porte au reflux pendant 12 heures, un mélange de 25 mmoles de dihydroxy-2,6 acétophénone, 26 mmoles de sulfate de méthyle, 75 mmoles de $K_2CO_3$ et 60 ml d'acétone. Puis on refroidit le milieu réactionnel, sépare les matières minérales par filtration et les rince au dichlorométhane. Après évaporation de la phase organique et flash chromatographie, on obtient le produit attendu de manière pratiquement quantitative (F = 55° C).

Dans les mêmes conditions opératoires, mais en utilisant le bromure de benzyle à la place de la dihydroxy-2,6 acétophénone, on obtient 1'hydroxy-2 benzyloxy-6 acétophénone [(XX), $R_{23}$ = $OCH_2$Ø ; numéro de code : 42] avec un rendement de 60 % (F = 109° C).

Exemple 2 : Bromo-3 hydroxy-2 méthoxy-6 acétophénone [(XIX) ; X = Br, $R_{23}$ = $OCH_3$]
Numéro de code : 10

Par action du brome sur le composé de numéro de code 9 préparé à l'exemple précédent, dans le chlorure de méthylène, selon la technique décrite par C.L. COON, W.G. BLUTCHER, M.E. HILL, J. Org. Chem. 38, 4243 (1973), on obtient le composé attendu avec un rendement de 76 % (F = 101° C).

De la même manière, mais en partant du composé de numéro de code 42, on obtient la bromo-3 hydroxy-2 benzyloxy-6 acétophénone [(XIX), $R_{23}$ = $OCH_2$Ø, X = Br ; numéro de code : 43] avec un rendement de 84 % (F = 124° C ; $IR_{KBr}$ : bande CO à 1620 $cm^{-1}$).

Exemple 3 : Hydroxy-2 iodo-3 méthoxy-6 acétophénone [(XIX) ; $R_{23}$ = $OCH_3$, X = I]
Numéro de code : 13

On solubilise 409 g (2,45 moles) de l'acétophénone de numéro de code 9 préparé à l'exemple 1, dans 3 litres d'acide acétique cristallisable. Parallèlement, on solubilise 420 g (2,60 moles) de chlorure d'iode dans 400 ml d'acide acétique. A 17° C, on ajoute, en 15 minutes, la solution de chlorure d'iode à la solution de l'acétophénone. Après 10 minutes d'agitation, la réaction est achevée. On verse ensuite le mélange obtenu sur 10 litres d'eau froide et agite pendant 30 minutes, puis filtre, essore, rince avec 2 litres d'eau, essore, sèche 1 heure 30 minutes en étuve ventilée à 50° C et recristallise le produit dans l'éthanol pour isoler 634 g (Rendement = 88 %) du composé attendu.

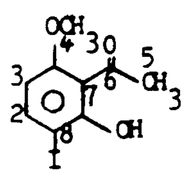

| | |
|---|---|
| . F = | 115° C |
| . $IR_{KBr}$ : | bande CO à 1615 $cm^{-1}$ |
| . RMN $^{13}C$ ($CDCl_3$, TMS) : | déplacements en ppm |
| | $C_2$ = 144,7 ; $C_3$ = 103,8 ; $C_4$ = 55,9 ; |
| | $C_5$ = 33,2 ; $C_6$ = 204,7 ; $C_7$ = 115,5 ; |
| | $C_8$ = 75,7. |

Exemple 4 : Hydroxy-2 allyloxy-3 méthoxy-6 acétophénone [(V), $R_{12}$ = allyloxy, $R_{22}$ = $OCH_3$]
Numéro de code : 15

Sous balayage d'azote, on recouvre 0,1 ml d'hydrure de sodium dispersé à 80 % dans l'huile par 30 ml de DMF. On ajoute goutte à goutte une solution de 0,12 mole d'alcool allylique dans 20 ml de DMF, sans dépasser 30° C. En fin d'addition, on agite 1 heure à 20° C. On ajoute ensuite 0,025 mole du composé de

numéro de code 13 préparé à l'exemple 3 et 0,0026 mole de CuI, puis agite 1 heure à environ 80° C. En fin de réaction, on verse sur 150 ml d'eau, ajuste le pH à 3 avec de l'acide chlorhydrique dilué, extrait par l'éther éthylique, sèche la phase organique sur $Na_2SO_4$, évapore à sec et purifie par flash chromatographie [éluant : heptane (85) - acétate d'éthyle (15)]. On isole ainsi le composé attendu avec un rendement de 45 %.

Par un procédé similaire, mais à partir des réactifs appropriés et en utilisant 0,035 mole de $Cu_2O$ à la place de 0,0025 mole de CuI quand on met en oeuvre un alkylthioalcool à la place de l'alcool allylique, on obtient les autres composés (V) rassemblés dans le tableau I ci-après, ainsi que l'hydroxy-2 méthoxy-3 benzyloxy-6 acétophénone [(V), $R_{12}$ = $OCH_3$, $R_{22}$ = $OCH_2Ø$] de numéro de code 44 (F = 103° C : IR-(KBr) : bande CO à 1625 $cm^{-1}$)

## TABLEAU I

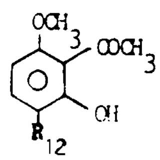

(V)

Données spectrales communes des composés (V) :

- IR (KBr) : bande CO à 1620 $cm^{-1}$
- RMN $^1H$ ($CDCl_3$), TMS) :
  . 6,9 et 6,3 ppm, 2d (2H), Ar-H
  . vers 13 ppm, 1s (1H), O-H
  . 3,8 ppm, 1s (3H), O-$CH_3$
  . 2,6 ppm, 1s (3H), $COCH_3$

| Numéro de Code | $R_{12}$ | Point de fusion (°C) | RMN $^1H$ ($CDCl_3$, TMS) des protons de $R_{12}$ |
|---|---|---|---|
| 7b | $CH_3CH_2O$- | 70 | 4ppm,q(2H),$OCH_2CH_3$;3ppm,t(3H), $OCH_2CH_3$ |
| 14 | $CH_3(CH_2)_2O$- | 90 | 3,9ppm,m(2H),$OCH_2R$;1,7ppm,m(2H), $OCH_2CH_2CH_3$;1ppm,m(3H),$OCH_2CH_2CH_3$ |
| 15 | $CH_2$=$CHCH_2O$ - | Huile | de 5 à 6ppm,m(3H),$CH_2$=CH;4,5ppm,d(2H), $CH_2$=CH-$CH_2O$ |
| 16 | $ØCH_2O$ | " | 7,3ppm,m(5H),ArH;5ppm,s(2H),$OCH_2Ar$ |
| 17 | $CH_3O(CH_2)_2O$- | 63 | 4ppm,t(2H),$ArOCH_2CH_2R$ ; 3,7ppm,m(5H), $ArOCH_3$+-$CH_2OCH_3$;3,4ppm,s(3H),$ROCH_3$ |
| 18 | $cC_6H_{11}O$- | Huile | 3,9ppm,s+t(4H),$OCH_3$ + ArOCHRR'; 1,5ppm,m(10H,$CH_2$cyclohexyl |
| 21 | $CH_3S$- | 83 | 7,3ppm,d(1H),(J:8Hz)ArH;2,3ppm,s(3H), $CH_3S$ |
| 22 | $CH_3CH_2S$- | 57 | 7,4ppm,d(1H),(J:8Hz)ArH;2,8ppm,q(2H), $CH_3CH_2S$ ; 1,2ppm,d(3H),$CH_3CH_2S$ |

Exemple 5 : (Pipéridino-2 éthoxy)-2 méthoxy-3 benzyloxy-6 acétophénone [(VII) ; n = 2, p = 5]
Numéro de code : 45

On porte, au reflux pendant 2 heures, un mélange de 0,15 mole du composé de numéro de code 44 préparé conformément à l'exemple 4, de 0,15 mole de chlorhydrate de N-(chloro-2 éthyl)-pipéridine, de 0,46 mole de $K_2CO_3$ et de 750 ml d'acétonitrile. Puis on refroidit, filtre, évapore à sec, reprend le résidu par 300 ml d'éther éthylique, lave la phase organique par 50 ml d'eau, sèche sur $Na_2SO_4$ et évapore. Après purification selon les techniques usuelles, on obtient le composé attendu avec un rendement de 87 % ($IR_{KBr}$ : bande CO à 1710 $cm^{-1}$).

Exemple 6 : (Pipéridino-2 éthoxy)-2 méthoxy-3 hydroxy-6 acétophénone [(VI) ; n = 2, p = 5]
Numéro de code : 46

A une solution de 30 mmoles du composé de numéro de code 45 préparé à l'exemple 5 dans 100 ml d'éthanol, on ajoute 2 g de palladium à 10 % sur charbon contenant 50 % d'humidité et des traces d'éthanol chlorhydrique. On agite 2 heures à 20° C sous atmosphère d'hydrogène. Puis on filtre, rince le catalyseur à l'éthanol et évapore à sec pour obtenir le composé attendu, avec un rendement de 82 %, sous la forme d'une huile pure ($IR_{KBr}$ : bande CO à 1640$cm^{-1}$).

Exemple 7 : O-alcoylation en 6 de l'acétophénone de numéro de code 46 préparé à l'exemple 6 pour obtenir les composés de formule (IV) où $R_{11}$ = $OCH_3$

On chauffe au reflux un mélange de 20 mmoles de l'acétophénone 46, 22 mmoles d'un composé de formule $R_{210}$-Y définie dans le tableau II ci-après, 60 mmoles de $K_2CO_3$ et 70 ml d'acétonitrile. La réaction une foie achevée (4-120 heures), on évapore à sec, reprend à l'eau, extrait à l'éther éthylique, extrait le chlorhydrate de la phase organique par HCl 1N, lave la phase aqueuse a l'éther éthylique, relargue l'acétophénone O-alcoylée de la phase aqueuse par $NH_4OH$, extrait à l'éther éthylique, sèche sur $MgSO_4$, évapore à sec et filtre l'huile obtenue sur silice (éluant : dichlorométhane puis acétate d'éthyle), cette huile correspondant aux composés (IV) attendus. Un certain nombre de ces composés sont rassemblés dans le tableau II ci-après.

## TABLEAU II

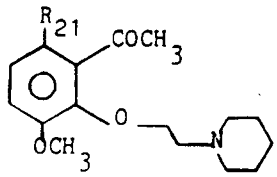

(IV)

Données spectrales communes des composés (IV) :

- IR (KBr) : bande CO à 1710 $cm^{-1}$
- RMN $^1H$ ($CDCl_3$, TMS) :
  - . 6,6 ppm, q (2H), Ar-H
  - . 4,1 ppm, t (2H), $O-CH_2-CH_2-N$
  - . 3,7 ppm, s (3H), $O-CH_3$
  - . 2,5 ppm, m + s (9H), $CO-CH_3$, 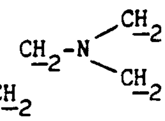
  - . 1,5 ppm, m (6H), 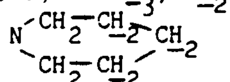

| Numéro de Code | $R_{21}$ | $R_{210}$-Y | RMN $^1H$ ($CDCl_3$), TMS) des protons de $R_{21}$ |
|---|---|---|---|
| 47 | $CH_3(CH_2)_3O$ | I | 4ppm, m (4H) dont $RCH_2O$; 1,5ppm,m (10H) dont $CH_3(CH_2)_3O$; 1ppm, m (3H), $CH_3$ |
| 48 | $CH_3(CH_2)_2O$ | Br | 3,9ppm, m (4H) dont $RCH_2O$ ; 1,5ppm, m (8H) dont $CH_3CH_2CH_2O$ ; 1ppm,m (3H), $CH_3$ |
| 49 | $(CH_3)_2CHO$ | Br | 4,3ppm, m (1H), $(CH_3)_2CHO$ ; 1,3ppm, d (6H), $(CH_3)_2CHO$ |
| 50 | $CH_3(CH_2)_7O$ | OMs | 3,8ppm,m(4H) dont $OCH_2R$ ; 1,4ppm,m, (21H) dont $OCH_2(CH_2)_6CH_3$ |
| 51 | $cC_6H_{11}O$ | Oms | 4,1ppm,m(3H) dont $CH(CH_2)_5$ ; 1,5ppm, m(16H) dont $CH(CH_2)_5$ |
| 52 | $CH_3CH_2O$ | $OSO_3Et$ | 4ppm,m(4H) dont $CH_3CH_2O$ ; 1,3ppm, m(9H) dont $CH_3CH_2O$ |

Exemple 8 : Préparation des 3acétophénones de formule (IV) où $R_{21}$ = $OCH_3$

Le mode opératoire utilisé est celui décrit dans le cadre de l'exemple 5, mais en utilisant à titre de produits de départ, les composés (V) où $R_{22}$ = $OCH_3$.

Un certain nombre des composés ainsi synthétisés (qui se présentent tous sous la forme d'huile) sont rassemblés dans le tableau III ci-après.

**TABLEAU III**

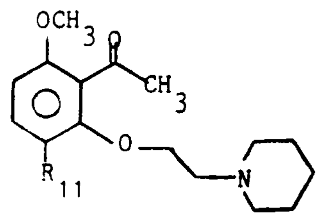

(IV)

| Numéro de code | $R_{11}$ | Produits de départ | Remarques - Traitements particuliers |
|---|---|---|---|
| 100 | $CH_3CH_2O$ | 7b | --- |
| 101 | $CH_3(CH_2)_2O$ | 14 | --- |
| 102 | $CH_2=CHCH_2O$ | 15 | Purification par recristallisation de l'oxalate dans l'acétone |
| 103 | $\phi CH_2O$ | 16 | Chlorhydrate F = 180° C |
| 104 | $CH_3O(CH_2)_2O$ | 17 | --- |
| 105 | $cC_6H_{11}O$ | 18 | --- |
| 106 | $CH_3S$ | 21 | Chromatographie $CH_2Cl_2$ (95) MeOH (4,5) $NH_4OH$ (0,5) |
| 107 | $CH_3CH_2S$ | 22 | Chromatographie $CH_2Cl_2$ (95) MeOH (4,5) $NH_4OH$ (0,5) |

Il est à noter également que pour ces composés :

- en PMN $^1$H, les pics correspondants à la chaîne éther aminé apparaissent à 4 ppm, t (2H),

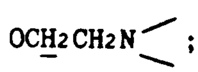

2,5 ppm, m (6H),

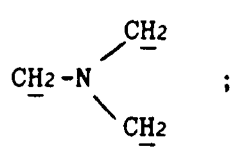

et 1,5 ppm, m (6H),

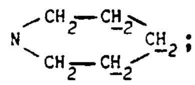

et

- en IR, la bande CO apparaît à 1680-1700 $cm^{-1}$

Exemple 9 : Acide diméthoxy-2,3 bromo-6 benzoïque (XV)
Numéro de code : 72

On solubilise 91 g (0,5 mole) d'acide diméthoxy-2,3 benzoïque (XVI) dans 1,2 litre de soude 1,6 N, glace à 0° C, ajoute 38,4 ml (0,75 mole) de brome et agite 30 minutes à + 5° C.

Puis on acidifie par HCl, extrait avec l'éther éthylique et lave la phase organique au thiosulfate de sodium, sèche et l'évapore. on reprend le résidu dans 200 ml d'éthanol, ajoute 3 gouttes d'acide sulfurique et laisse refluer une nuit. Puis on évapore à sec, reprend le résidu par 300 ml d'éther éthylique, extrait la phase aqueuse par 300 ml de soude 2N, lave la phase aqueuse à l'éther éthylique avant de la sécher sur $Na_2SO_4$ en présence de noir animal. Puis on filtre, évapore et sèche à la pompe à palette. On isole ainsi 107 g du composé attendu pur, cristallisable dans le mélange éther de pétrole (50) - toluène (50).

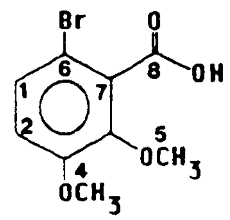

| | |
|---|---|
| . F = | 87° C |
| . $IR_{KBr}$ : | bande CO à 1710 $cm^{-1}$ |
| . Spectre de masse : | pic moléculaire à 261 |
| . RMN $^{13}C$ ($CDCl_3$) : | |
| | $C_1$ = 128,1 |
| | $C_2$ = 115 |
| | $C_4$ = 56,2 |
| | $C_5$ = 61,9 |
| | $C_6$ = 108,7 |
| | $C_7$ = 118,3 |
| | $C_8$ = 170,6 |

Exemple 10 : Chlorure de l'acide diméthoxy-2,3 bromo-6 benzoïque (XIV)
Numéro de code : 73

On agite à 20° C 6,8 mmoles de l'acide de numéro de code 72 préparé à l'exemple précédent, avec 34 mmoles de chlorure de thionyle dans 5 ml de toluène pendant 48 heures. Puis on évapore à sec et élimine l'excès de chlorure de thionyle par distillation azéotropique du toluène. Le composé résultant attendu est utilisé tel quel dans le procédé de l'exemple 11.

Exemple 11 : Diméthoxy-2,3 méthyl-6 acétophénone (XIII)
Numéro de code : 75

On disperse 0,250 mole de $MnI_2$ dans 800 ml d'éther éthylique fraichement distillé sur $LiAlH_4$. A 0° C, on ajoute en 15 minutes 0,24 mole de méthyllithium (solution 1,6 N dans l'éther éthylique), puis on agite ½ heure à 0° C, puis 2 heures à température ambiante.

Ensuite, on refroidit le milieu réactionnel à 0° C et ajoute 0,12 mole du composé de numéro de code 73 préparé à l'exemple 10 et dilué par 50 ml d'éther éthylique. Après réaction pendant ½ heure à 0° C, puis pendant 2 heures 30 à 25° C, on ajoute 120 ml d'ammoniaque 2N, agite 15 minutes, neutralise à

l'acide chlorhydrique, extrait à l'éther éthylique, lave la phase organique avec une solution de thiosulfate, puis sèche et évapore à sec (Rendement : 88 %).

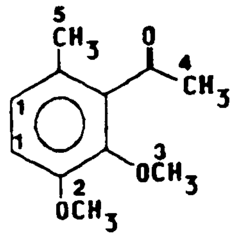

. IR(KBr) : bande CO à 1700 $cm^{-1}$

. RMN $^{13}C$ $(CDCl_3)$ :
$C_1$ = 113,3 - 125,9
$C_2$ = 56
$C_3$ = 61,5
$C_4$ = 32,3
$C_5$ = 18,2
$C_6$ = 205

Exemple 12 : Acide diméthoxy-2,3 éthyl-6 benzoîque (XVIII)
Numéro de code : 94

On dissout 13 g (50 mmoles) de l'acide (XV) de numéro de code 72 dans 160 ml de THF à - 100° C, et on ajoute lentement 67 ml (107 mmoles) d'une solution de n-butyllithium 1,6 N. on laisse en agitation 15 mn et ajoute 37,6 ml (500 mmoles) d'iodure d'éthyle. On laisse 1 heure à - 90° C puis 1 heure à - 60° C et 1 heure à 20° C. Le milieu réactionnel est versé sur HCl 2N. La phase aqueuse est extraite à l'éther éthylique. La phase organique est séchée et concentrée. Le résidu est repris dans l'éthanol avec quelques gouttes d'$H_2SO_4$ et chauffée au reflux une nuit et concentrée. Le résidu est repris par le mélange NaOH (2N) - éther éthylique. Après extraction, la phase aqueuse est acidifiée par HCl et extraite à l'éther éthylique. Après séchage de la phase organique suivi de sa concentration, on récupère 8,24 g du produit attendu sous forme d'huile.

. IR(KBr) : bande CO : 1700 $cm^{-1}$
bande OH : 3100 $cm^{-1}$

Exemple 13 : Chlorure d'acide diméthoxy-2,3 éthyl-6 benzoîque (XVII)
Numéro de code : 97

Ce composé est préparé selon un mode opératoire analogue à celui de l'exemple 10, mais à partir du composé de numéro de code 94.

. Rendement : 77 %
. IR (KBr) : bande CO à 1795 $cm^{-1}$

Exemple 14 : Diméthoxy-2,3 éthyl-6 acétophénone (XIII)
Numéro de code : 98

Ce composé est préparé selon un mode opératoire analogue à celui de l'exemple 11, mais à partir du composé de numéro de code 97.

. IR (KBr) : bande CO à 1705 $cm^{-1}$

Exemple 15 : Hydroxy-2 méthoxy-3 méthyl-6 acétophénone (XII)
Numéro de code : 92

A une solution de 8,32 g (43mmoles) du composé de numéro de code 75 préparé à l'exemple 11, dans 100 ml de $CH_2Cl_2$ à 0° C, on ajoute 5,7 g (43 mmoles) de $AlCl_3$, agite 1 heure à 0° C, puis 6 heures à reflux. Puis on refroidit le mélange réactionnel et le verse sur 50 ml d'acide chlorhydrique concentré, extrait à l'acétate d'éthyle, lave à l'eau la phase organique, puis la sèche sur $Na_2SO_4$ et l'évapore à sec. Après

purification par chromatographie, on récupère 5,93 g d'une huile jaune pure qui correspond au composé attendu.

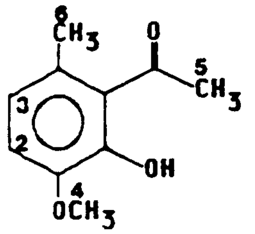

. IR(KBr) : - bande CO à 1690 $cm^{-1}$
- bande OH à 3400 $cm^{-1}$

. RMN $^{13}C$ ($CDCl_3$) : $C_2$-$C_3$ = 113,5-121,6
$C_4$ = 56,2
$C_5$ = 32,5
$C_6$ = 21,2

Exemple 16 : (Pipéridino-2 éthoxy)-2 méthoxy-3 méthyl-6 acétophénone [(IV) ; n = 2, p = 5, $R_{11}$ = $OCH_3$, $R_{21}$ = $CH_3$]
Numéro de code : 108

Ce composé est préparé selon un mode opératoire analogue à celui de l'exemple 5, mais à partir du composé de numéro de code 92 préparé à l'exemple 15.

Les spectres IR et RMN $^1H$ du composé en cause présentent les caractéristiques mentionnées dans le cadre de l'exemple 8.

Exemple 17 : [(Tétrahydropyranyl-2-oxy)-2 éthoxy]-2 benzyloxy-3 méthoxy-6 acétophénone (XI)
Numéro de code : 31

On chauffe au reflux pendant 5 jours dans l'acétonitrile, 5,44 g (20 mmoles) du composé de numéro de code 16 préparé conformément à l'exemple 4, 4,8 g (29 mmoles) de (chloro-2 éthoxy)-2 tétrahydropyrane et 8,3 g (60 mmoles) de $K_2CO_3$. Puis on évapore à sec, reprend le résidu par 25 ml d'eau, extrait avec 35 ml d'éther éthylique, sèche et évapore après filtration sur silice (éluant : dichlorométhane). On récupère 3,95 g du composé attendu qui se présente sous la forme d'une huile.

. IR(KBr) : bande CO à 1710 $cm^{-1}$

Exemple 18 : [(Tétrahydropyranyl-2-oxy)-2 éthoxy]-2 hydroxy-3 méthoxy-6 acétophénone (X)
Numéro de code : 32

On agite 3,95 g (10 mmoles) du composé de numéro de code 31 obtenu à l'exemple précédent, avec 0,8 g de Pd à 10 % sur charbon contenant 50 % d'eau sous une atmosphère d'hydrogène à 20° C. Après une nuit de contact, on sépare le catalyseur par filtration, le rince à l'éthanol et évapore à sec. On isole ainsi 3,05 g du composé attendu.

. F = 83° C
. IR(KBr) = - bande CO à 1700 $cm^{-1}$
- bande OH à 3300 $cm^{-1}$

Exemple 19 : (Hydroxy-2 éthoxy)-2 (trifluoro-2,2,2 éthoxy)-3 méthoxy-6 acétophénone [(IX), $R_{110}$ = $OCH_2CF_3$]
Numéro de code : 33a

On chauffe au reflux le mélange de 5,75 g (18,5 mmoles) du composé de numéro de code 32 préparé à l'exemple précédent, 8,6 g (34,4 mmoles) de triflate de trifluoroéthyle, 7,68 g (55,6 mmoles) de $K_2CO_3$ et 60 ml d'acétone. Après 3 heures de contact, on filtre, évapore à sec, reprend par un mélange éthanol chlorhydrique 3N (25 ml) - eau (25 ml). Après agitation puis neutralisation au bicarbonate de sodium, on évapore à sec, reprend le résidu d'évaporation avec 50 ml d'eau, extrait la phase aqueuse à l'éther

éthylique puis sèche et évapore à sec la phase organique. On obtient ainsi 4,47 g du composé attendu sous la forme d'une huile pure.

IR(KBr) : - bande OH à 3430 $cm^{-1}$
- bande CO à 1700 $cm^{-1}$

Exemple 20 : (Hydroxy-2 éthoxy)-2 isopropyloxy-3 méthoxy-6 acétophénone [(IX), $R_{110}$ = $OCH(CH_3)_2$]
Numéro de code : 33b

On dissout 5.7 g (18,5 mmoles) du composé de numéro de code 32 obtenu à l'exemple 18, dans 60 ml de DMF. On ajoute 2,23 g (56 mmoles) de NaOH en poudre, tiédit à 50° C, ajoute 3,5 ml (37 mmoles) de bromure d'isopropyle. Après 2 heures 30 à 50° C, on verse le mélange réactionnel dans 200 ml d'eau et extrait par deux fois 200 ml d'éther éthylique. On agite les phases éthérées quelques minutes avec 15 ml d'acide chlorhydrique concentré, ensuite on lave à l'eau puis avec une solution de bicarbonate de sodium, sèche et évapore. On isole ainsi 4,97 g d'une huile pure qui correspond au composé attendu.

IR(KBr) : - bande OH à 3350 $cm^{-1}$
- bande CO à 1700 $cm^{-1}$

Exemple 21 : (Mésyloxy-2 éthoxy)-2 (trifluoro-2,2,2 éthoxy)-3 méthoxy-6 acétophénone [(VIII), $R_{110}$ = $CF_3CH_2O$]
Numéro de code : 34a

On dissout 14 mmoles du composé de numéro de code 33a dans 50 ml de dichlorométhane. On ajoute 21 mmoles de triéthylamine, glace à 0° C et ajoute très lentement 15 mmoles de chlorure de mésyle. En fin d'addition, on agite 30 minutes à 0° C, puis on lave à l'eau, sèche sur $Na_2SO_4$ et évapore à sec. on isole ainsi le composé attendu.

. IR(KBr) : bande CO à 1710 $cm^{-1}$

Par le même procédé, mais à partir du composé de numéro de code 33b, on obtient la (mésyloxy-2 éthoxy)-2 isopropoxy-3 méthoxy-6 acétophénone dont le numéro de code est 34b [(VIII), $R_{110}$ = OiPr]

Exemple 22 : (Pipéridino-2 éthoxy)-2 (trifluoro-2,2,2 éthoxy)-3 méthoxy-6 acétophénone [(IV), n = 2, p = 5, $R_{11}$ = $OCH_2CF_3$, $R_{21}$ = $OCH_3$]
Numéro de code : 35a

On porte à ébullition (12,9 mmoles) du composé de numéro de code 34a préparé à l'exemple précédent dans 500 mmoles de pipéridine. Ensuite, on évapore à sec, reprend par l'acétate d'éthyle, extrait par HCl 2N, lave à l'éther éthylique la phase aqueuse, relargue le produit de cette phase par addition de $NH_4OH$, puis extrait à l'acétate d'éthyle. On lave plusieurs fois à l'eau la phase organique puis on la sèche sur $Na_2SO_4$, la filtre et l'évapore. On obtient ainsi le composé attendu sous la forme d'une huile.

. RMN $^1H$ ($CDCl_3$, TMS) : 6,7 ppm, q(2H), Ar-H : 4,3 ppm, m (4H), Ar-O-$CH_2$-$CF_3$ et Ar-O-$CH_2CH_2$-N ; 3,7 ppm, s (3H), $OCH_3$ ; 2,5 ppm, m (9H), CO-$CH_3$ et

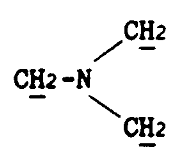 ;

1,5 ppm, m (6H),

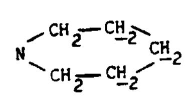

De la même manière, mais à partir du composé de numéro de code 34b, on obtient la (pipéridino-2 éthoxy)-2 isopropory-3 méthoxy-6 acétophénone [(IV), n = 2, p = 5, $R_{11}$ = OiPr, $R_{21}$ = $OCH_3$] et dont le numéro de code est 35b.

. RMN $^1H$ ($CDCl_3$, TMS) : 6,7 ppm, q (2H), Ar-H ; 4,2 ppm, t + m (3H), $ArOCH_2CH_2N$ + ArOCH-

$(CH_3)_2$ ; 1,4 ppm, d + m (12H), $(C\underline{H}_2)_3$ + $CH(C\underline{H}_3)_2$

Exemple 23 : [(Pipéridino-2 éthoxy) -2 allyloxy-3 méthoxy-6 phényl]-1 (hydroxy-4 phényl)-3 propen-2 one-1 [(III), n = 2, p = 5, $R_{11}$ = allyloxy, $R_{21}$ = $OCH_3$, $R_3$ = H]
Numéro de code : 125

On solubilise 7,7 g (23 mmoles) du composé du numéro de code 102 préparé conformément à l'exemple 8, dans 100 ml d'éthanol, ajoute 3,1 g (25 mmoles) d'hydroxy-4 benzaldéhyde et 4,6 g (110 mmoles) de NaOH aqueuse à 50 %, puis agite à 20° C durant 3 jours. Ensuite, on évapore l'alcool, reprend à l'eau, lave à l'éther isopropylique, acidifie avec l'acide chlorhydrique (le chlorhydrate précipite), lave la phase aqueuse hétérogène à l'éther isopropylique, traite la phase aqueuse par 50 ml de $NH_4OH$ 5N, extrait à l'acétate d'éthyle, puis sèche la phase organique, l'évapore et cristallise le produit obtenu dans 50 ml d'acétate d'éthyle. on isole ainsi 6,03 g du composé attendu.

. F = 132° C
. IR(KBr) : - bande CO à 1650 $cm^{-1}$
- bande OH à 3400 $cm^{-1}$

Par mise en oeuvre du même mode opératoire, mais en partant des réactifs appropriés, on obtient les composés de numéros de code 120, 122 et 126 figurant dans le tableau IV ci-après.

Exemple 24 : [(Pipéridino-2 éthoxy)-2 méthoxy-3 benzyloxy-6 phényl]-1 {[(tétrahydropyranyl-2) oxy]-4 phényl}-3 propen-2 one-1 [(III), n = 2, p = 5, $R_{11}$ = $OCH_3$, $R_{21}$ = $OCH_2Ø$, $R_3$ = tétranydropyranyl-2]
Numéro de code : 114

On dissout 56,6 g (148 mmoles) du composé de numéro de code 45 préparé à l'exemple 5 et 30 g (148 mmoles) de (tétrahydropyranyl-2)oxy-4 benzaldéhyde dans 600 ml d'alcool éthylique. On ajoute ensuite 37 ml (370 mmoles) de NaOH aqueuse 10 N, agite 5 heures à 25° C, concentre l'alcool au 3/4, verse le mélange réactionnel dans 1 litre d'eau, extrait avec deux fois 200 ml de $CH_2Cl_2$, lave la phase organique par trois fois 200 ml d'eau, sèche et évapore. On cristallise le produit attendu dans l'éther éthylique et isole ainsi 65 g du composé attendu.

. F = 103° C
. IR(KBr) : bande CO à 1650 $cm^1$

Par mise en oeuvre du même mode opératoire, mais à partir des réactifs appropriés, on obtient les composés de numéros de code 110, 114 à 119, 124 et 127 à 131 figurant dans le tableau IV ci-après.

TABLEAU IV

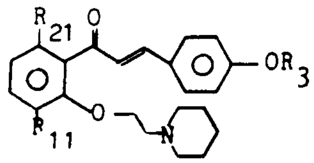 (III)

| No. de Code | $R_{11}$ | $R_{21}$ | $R_3$ | No. de Code du produit de départ | Point de fusion (°C) | Traitements de purification |
|---|---|---|---|---|---|---|
| 110 | $CF_3CH_2O$ | $CH_3O$ | H | 35a | Huile | Filtré sur silice |
| 114 | $CH_3O$ | BzO | THP | 45 | 103 | Crist. dans $Et_2O$ |
| 115 | $CH_3O$ | $CH_3(CH_2)_3O$ | Bz | 47 | Huile | --- |
| 116 | $CH_3O$ | $CH_3(CH_2)_2O$ | Bz | 48 | Huile | --- |
| 117 | $CH_3O$ | $(CH_3)_2CHO$ | Bz | 49 | Huile | --- |
| 118 | $CH_3O$ | $CH_3(CH_2)_7O$ | Bz | 50 | Huile | --- |
| 119 | $CH_3O$ | $cC_6H_{11}O$ | Bz | 51 | Huile | --- |
| 120 | $CH_3O$ | $CH_3CH_2O$ | H | 52 | 124 | Crist. dans $Et_2O$ |
| 122 | $CH_3CH_2O$ | $CH_3O$ | H | 100 | 153 | Crist. dans $Et_2O$ |
| 124 | $CH_3(CH_2)_2O$ | $CH_3O$ | Bz | 101 | Huile | --- |
| 125 | $CH_2{=}CH{-}CH_2O$ | $CH_3O$ | H | 102 | 132 | Crist. dans AcOEt |
| 126 | BzO | $CH_3O$ | H | 103 | 137 | Crist. dans AcOEt |
| 127 | $CH_3O(CH_2)_2O$ | $CH_3O$ | Bz | 104 | Huile | Filtré sur silice |
| 128 | $cC_6H_{11}O$ | $CH_3O$ | Bz | 105 | Huile | Filtré sur silice |
| 129 | $CH_3S$ | $CH_3O$ | THP | 106 | 88 | Chromat. puis crist./$iPr_2O$ |
| 130 | $CH_3CH_2S$ | $CH_3O$ | THP | 107 | Huile | Filtré sur silice |
| 131 | $CH_3O$ | $CH_3$ | Bz | 108 | Huile | Filtré sur silice |
| - | $CH_3O$ | $C_2H_5$ | Bz | - | Huile | --- |

THP = tétrahydropyranyl-2

Bz = benzyl

Exemple 25 : Réduction des composés (III) pour lesquels $R_3$ = H, préparés à l'exemple précédent, pour obtenir les composés (I) correspondants

On dissout 10 mmoles de composé (III) avec $R_3$ = H, dans 150 ml d'éthanol, ajoute 50 mmoles de pyridine, 25 mmoles de NaOH 10 N et porte au reflux. Ensuite, on ajoute en 3 heures de 50 à 100 mmoles de borohydrure de sodium. En fin de réaction, on verse le mélange réactionnel sur 700 ml de HCl 0,5 N glacé, agite 30 minutes à 0° C, puis relargue le produit à l'ammoniaque, extrait au $CH_2Cl_2$, lave la phase organique à l'eau, sèche sur $Na_2SO_4$, évapore, purifie par chromatographie flash [éluant : $CH_2Cl_2$ (95) - $CH_3OH$ (4,5) - $NH_4OH$ (0,5)], cristallise dans l'acétate d'éthyle et recristallise si besoin est dans l'éthanol.

Par ce procédé, on a obtenu les composés de numéros de code 167 et 168 répertoriés dans le tableau V ci-après, respectivement à partir des composés de numéros de code 125 et 126.

Exemple 26 : Réduction des composés (III) pour lesquels $R_3$ = tétrahydropyranyl-2, préparés à l'exemple 24, pour obtenir les composés (I) correspondants

On chauffe au reflux 10 mmoles de composé (III) avec $R_3$ = tétrahydropyranyl-2, dans 100 ml d'éthanol contenant une goutte de NaOH, ajoute en 3 heures 50 mmoles de horohydrure de sodium, puis refroidit, évapore à sec, reprend par 150 ml d'acétate d'éthyle, verse sur 10 ml de HCl à 0° C, agite 15 minutes, puis relargue par l'ammoniaque, extrait par trois fois 120 ml d'acétate d'éthyle, lave la phase organique par une solution saturée de chlorure de sodium, sèche sur $Na_2SO_4$, évapore à sec et purifie par flash chromatographie [éluant : $CH_2Cl_2$ (97) - $CH_3OH$ (2,7) - $NH_4OH$ (0,3)].

Par ce procédé, on a obtenu les composés de numéros de code 146, 174 et 175 répertoriés dans le tableau V ci-après, respectivement à partir des composés de numéros de code 114, 129 et 130.

Exemple 27 : Réduction des composés (III) préparés à l'exemple 24, pour obtenir les composés (I) correspondants 1ère Etape : Préparation des composés (II)

On dissout 10 mmoles des composés (III) dans un mélange alcool éthylique (50 ml) - THF (50 ml), ajoute 20 % en poids de Pd à 10 % sur charbon contenant 50 % d'humidité, agite à 20° C sous atmosphère d'hydrogène. En fin de réaction, on sépare le catalyseur par filtration, le rince au THF, évapore à sec, recristallise dans un solvant approprié.

On obtient ainsi les composés (II) répertoriés dans le tableau VI ci-après.

2ème Etape : Réduction des composés (II) en composés (I) correspondants

On dissout 10 mmoles de composé (II) obtenu à l'étape précédente, dans 50 ml d'éthanol, ajoute 1 ml de NaOH 10 N (soit 10 mmoles), chauffe au reflux, ajoute ensuite en 3 heures, de 50 à 100 mmoles de borohydrure de sodium. En fin d'addition, on maintient 2 heures au reflux, refroidit, évapore à sec, reprend à l'acétate d'éthyle, verse sur 25 ml d'HCl 2N à 0° C, agite 15 minutes à 0° C, neutralise avec $NH_4OH$, extrait par deux fois 100 ml d'acétate d'éthyle, sèche, évapore à sec, chromatographie sur silice si nécessaire [éluant : $CH_2Cl_2$ (95) - $CH_3OH$ (4,5) - $NH_4OH$ (0,5)], cristallise et recristallise si besoin est.

On obtient ainsi les composés (I) répertoriés dans le tableau (V) autres que ceux de numéros de code 146, 167, 168, 174 et 175.

TABLEAU V

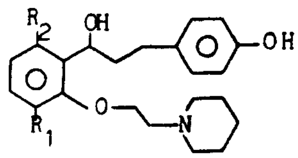

(I)

| Numéro de code | $R_1$ | $R_2$ | Point de fusion (°C) | Solvant de recristallisation |
|---|---|---|---|---|
| 137 | $CF_3CH_2O$ | $CH_3O$ | 117 | $Et_2O$ |
| 146 | $CH_3O$ | BzO | 153 | $CH_3OH$ |
| 148 | $CH_3O$ | OH | 153 | AcOEt |
| 150 | $CH_3O$ | $CH_3(CH_2)_3O$ | 123 | AcOEt |
| 152 | $CH_3O$ | $CH_3(CH_2)_2O$ | 124 | $Et_2O$ |
| 154 | $CH_3O$ | $(CH_3)_2CHO$ | 140 | $iPr_2O$ |
| 156 | $CH_3O$ | $CH_3(CH_2)_7O$ | 113 | $iPr_2O$ |
| 158 | $CH_3O$ | $cC_6H_{11}O$ | 157 | AcOEt |
| 160 | $CH_3O$ | $CH_3CH_2O$ | 148 | AcOEt |
| 164 | $CH_3CH_2O$ | $CH_3O$ | 119 | AcOEt |
| 166 | $CH_3(CH_2)_2O$ | $CH_3O$ | 99 | $iPr_2O$ |
| 167 | $CH_2{=}CH{-}CH_2{-}O$ | $CH_3O$ | 131 | AcOEt |
| 168 | BzO | $CH_3O$ | 148 | EtOH |
| 169 | OH | $CH_3O$ | 134 | AcOEt |
| 171 | $CH_3O(CH_2)_2O$ | $CH_3O$ | 110 | $Et_2O$ |
| 173 | $cC_6H_{11}O$ | $CH_3O$ | 142 | AcOEt |
| 174 | $CH_3S$ | $CH_3O$ | 155 | iPrOH |
| 175 | $CH_3CH_2S$ | $CH_3O$ | 117 | AcOEt |
| 177 | $CH_3O$ | $CH_3$ | 170 | $CH_2Cl_2$ |
| - | $CH_3O$ | $C_2H_5$ | 136 | $Et_2O$ |

TABLEAU VI

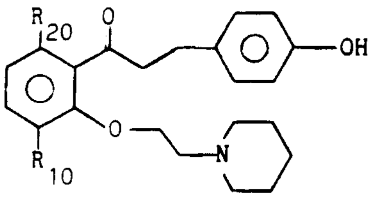

(II)

| Numéro de code | $R_{10}$ | $R_{20}$ | Point de fusion (°C) | Solvant de recristallisation |
|---|---|---|---|---|
| 136 | $CF_3CH_2O$ | $CH_3O$ | Huile | - |
| 147 | $CH_3O$ | OH | 104 | $C_2H_5OH$ |
| 149 | $CH_3O$ | $CH_3(CH_2)_3O$ | 131 | Acétone |
| 151 | $CH_3O$ | $CH_3(CH_2)_2O$ | 142 | " |
| 153 | $CH_3O$ | $(CH_3)_2CHO$ | 157 | " |
| 155 | $CH_3O$ | $CH_3(CH_2)_7O$ | Huile | - |
| 157 | $CH_3O$ | $cC_6H_{11}O$ | 107 | Ether isopropylique |
| 159 | $CH_3O$ | $CH_3CH_2O$ | 120 | " " |
| 163 | $CH_3CH_2O$ | $CH_3O$ | 115 | " " |
| 165 | $CH_3(CH_2)_2O$ | $CH_3O$ | Huile | - |
| 170 | $CH_3O(CH_2)_2O$ | $CH_3O$ | 100 | Ether isopropylique |
| 172 | $cC_6H_{11}O$ | $CH_3O$ | 154 | " éthylique |
| 176 | $CH_3O$ | $CH_3$ | Huile | - |
| - | $CH_3O$ | $C_2H_5$ | Huile | - |

Les composés (I) et leurs sels pharmacologiquement acceptables ont été étudiez chez l'animal de laboratoire et ont montré des activités pharmacologiques et notamment une activité antagoniste du calcium.

L'activité antagoniste du calcium a été mise en évidence par le test des contractions de l'artère basilaire isolée de lapin en milieu hyperpotassique selon K. Towart, S. Kazda dans Nimodipine Pharmacological Properties édité par P.E. Betz, K. Deck et F. Hoffmeister (1984), Ed. : E.K. Schattauer Verlag.

Le protocole de ce test est le suivant. Le lapin est anesthésié au pentobarbital à la dose de 30 mg/kg puis sacrifié par exanguination et décapité au niveau de la 3ème vertèbre cervicale. L'artère basilaire est prélevée (1,5 cm) entre les artères vertébrales et le polygone de Willis. L'artère basilaire est ensuite découpée en spirale sous un angle de 45°. Elle est fixée à l'aide d'une aiguille courbe entre un support en verre en forme de L et un capteur de déplacement (Grass, type FT03). Une tension mécanique de 500 mg est appliquée au vaisseau, avant de le laisser atteindre son état d'équilibre.

Pendant une période d'une heure, le vaisseau est rincé toutes les 20 minutes, jusqu'à obtention d'un niveau stable.

La contraction tonique consécutive à la dépolarisation provoquée par KCl est dépendante des mouvements calciques car elle est abolie par la suppression du calcium dans le milieu de survie.

On mesure les concentrations (en nM) qui réduisent de 50 % l'amplitude de la contraction induite par le KCl à 35 mmoles.

Les résultats obtenus avec certains composés selon l'invention dans le test précédant sont rassemblés, à titre d'exemple, dans le tableau VII ci-après qui donne par ailleurs la toxicité aiguë ($DL_{50}$) de certains des composés testés et qui est estimée chez la souris selon la méthode de J.T. LITCHFIELD et F. WILCOXON, J. Pharmacol. Exp. Ther. 1949, 96, 99.

TABLEAU VII

| Numéro de Code | $IC_{50}$ (nM) | $DL_{50}$ Souris (mg/kg/i.v.) |
|---|---|---|
| 169 | > 100 | |
| 164 | 24 | 17 |
| 137 | > 100 | |
| 166 | 120 | |
| 167 | 53 | |
| 171 | > 100 | |
| 173 | > 100 | |
| 168 | > 100 | |
| 174 | 27 | 22 |
| 175 | 31 | |
| 148 | > 100 | |
| 160 | 6 | 11,7 |
| 154 | 13 | |
| 152 | 10 | |
| 158 | > 100 | |
| 150 | 100 | 21,5 |
| 156 | > 100 | 27,5 |
| 146 | > 100 | |
| 177 | 30 | |

Compte tenu de ce qui précède, les composés (I) et de leurs sels pharmaceutiquement acceptables trouvent leur emploi en thérapeutique, humaine ou animale, pour le traitement des affections liées à une perturbation des mouvements intra et extra cellulaires du calcium au niveau cérébral et notamment des troubles du système cérébrovasculaire, tels que les hémorragies cérébrales, l'infarctus cérébral et la migraine.

Un autre objet de la présente invention consiste donc dans l'utilisation des composés selon l'invention pour la préparation de médicaments pour le traitement des affections susmentionnées et notamment des compositions pharmaceutiques qui comprennent au moins l'un de ces composés en association avec un support ou un excipient physiologiquement acceptable approprié pour lesdits composés.

Ces compositions peuvent par exemple être formulées en vue de leur administration par voie orale, parentérale ou rectale.

Pour l'administration par voie orale, lesdites compositions peuvent prendre la forme de comprimés, dragées ou gélules préparés par les techniques habituelles utilisant des supports et excipients connus tels que des agents liants, des charges, des lubrifiants et des agents de désintégration ; elles peuvent également prendre la forme de solutions, de sirops ou de suspensions.

Pour l'administration par voie parentérale, les compositions selon l' invention peuvent prendre la forme de solutions, suspensions ou émulsions injectables comprenant un véhicule liquide, huileux ou aqueux, parentéralement acceptable.

Enfin pour l'administration par voie rectale, les compositions selon l'invention peuvent prendre la forme de suppositoires comprenant les bases habituelles pour suppositoires.

La dose à laquelle les principes actifs, c'est-à-dire les composés selon l'invention, peuvent être administrées à l'homme ou à tout animal à sang chaud, dépend notamment de la voie d'admnistration, du poids corporel et de l'état pathologique du patient et de la puissance thérapeutique des composés mis en oeuvre. Généralement, par voie orale, les doses pourront atteindre 500 mg de principe actif par jour (en une ou plusieurs prises) ; par voie parentérale, les doses pourront atteindre 100 mg de principe actif par jour (en une ou plusieurs injections journalières) et par voie rectale, les doses pourront atteindre 300 mg de principe actif par jour (en un ou plusieurs suppositoires).

**Revendications**

**Revendications pour les Etats contractants suivants : AT, BE, CH, DE, FR, GB, IT, LI, LU, NL, SE**

**1.** Composés de formule générale :

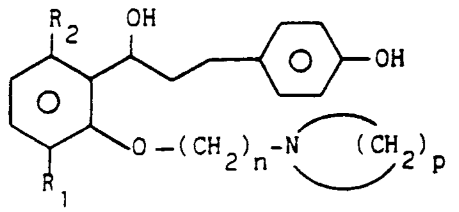

(I)

dans laquelle :

- $R_1$ et $R_2$ sont tels que :
  . soit $R_1$ représente un groupe méthoxy auquel cas $R_2$ est un groupe choisi parmi les suivants : hydroxyle ; alkyle en $C_1$-$C_4$ ; alkyloxy en $C_2$-$C_8$ ; cycloalkyloxy en $C_5$-$C_7$ ; benzyloxy ;
  . soit $R_2$ représente un groupe méthoxy auquel cas $R_1$ représente un groupe choisi parmi les suivants : hydroxyle ; alkyloxy en $C_2$-$C_8$ ; alkyloxy en $C_2$-$C_8$ substitué par un groupe méthoxy ; trifluoroalkyloxy en $C_1$-$C_4$ ; alcényloxy en $C_3$-$C_4$ ; cycloalkyloxy en $C_5$-$C_7$ ; benzyloxy ; alkylthio en $C_1$-$C_4$ ;
- n = 2 ou 3 ; et
- p = 4, 5 ou 6,

ainsi que leurs sels d'addition avec les acides minéraux ou organiques pharmaceutiquement acceptables.

**2.** Composés et sels selon la revendication 1, pour lesquels $R_1$ = méthoxy et $R_2$ est un groupe choisi parmi les suivants : benzyloxy, hydroxyle, n-butyloxy, n-propyloxy, i-propyloxy, n-octyloxy, cyclohexyloxy, éthoxy, méthyle, éthyle.

**3.** Composés et sels selon la revendication 1, pour lesquels $R_2$ = méthoxy et $R_1$ est un groupe choisi parmi les suivants : trifluoro-2,2,2 éthoxy, éthoxy, n-propyloxy, allyloxy, benzyloxy, hydroxyle, méthoxy-2 éthoxy, cyclohexyloxy, méthylthio, éthylthio.

**4.** Utilisation des composés et sels pharmaceutiquement acceptables selon la revendication 1, 2 ou 3, pour la préparation d'un médicament.

**5.** Composition pharmaceutique, caractérisée en ce qu'elle comprend, à titre de principe actif, au moins un composé ou sel selon la revendication 1, 2 ou 3 et un excipient physiologiquement acceptable.

**6.** Procédé de préparation des composés (I) et sels selon la revendication 1, 2 ou 3, caractérisé en ce qu'il comprend la réduction des composés de formule :

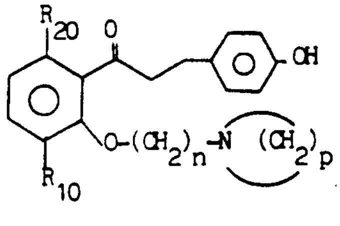

(II)

dans laquelle $R_{10}$ et $R_{20}$ ont respectivement la même signification que $R_1$ et $R_2$ dans la formule (I) définie à la revendication 1, à l'exception du groupe benzyloxy, n = 2 ou 3 et p = 4, 5 ou 6, cette réduction étant éventuellement suivie de la salification par un acide minéral ou organique pharmaceutiquement acceptable des composés (I) ainsi obtenus.

7. Procédé selon la revendication 6, caractérisé en ce que la réduction est réalisée par le borohydrure de sodium en milieu alcoolique, en présence de NaOH.

8. Procédé de préparation des composés (I) et sels selon la revendication 1, 2 ou 3, caractérisé en ce qu'il comprend la réduction des composés de formule :

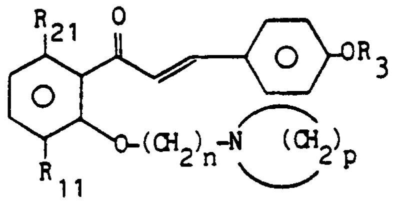

(III)

dans laquelle $R_{11}$ et $R_{21}$ ont respectivement la même signification que $R_1$ et $R_2$ dans la formule (I) définie à la revendication 1, à l'exception du groupe hydroxyle, $R_3$ = H, n = 2 ou 3 et p = 4, 5 ou 6, cette réduction étant éventuellement suivie de la salification par un acide minéral ou organique pharmaceutiquement acceptable des composés (I) ainsi obtenus.

9. Procédé selon la revendication 8, caractérisé en ce que la réduction est réalisée par le borohydrure de sodium, en milieu éthanolique et en présence de pyridine et de NaOH.

10. Procédé de préparation des composés (I) et sels selon la revendication 1, 2 ou 3, caractérisé en ce qu'il comprend la réduction des composés de formule :

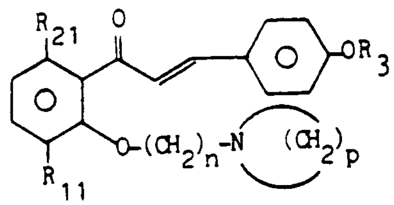

(III)

dans laquelle $R_{11}$ et $R_{21}$ ont respectivement la même signification que $R_1$ et $R_2$ dans la formule (I) définie à la revendication 1, à l'exception du groupe hydroxyle, $R_3$ = tétrahydropyranyl-2, n = 2 ou 3 et p = 4, 5 ou 6, puis l'hydrolyse acide des composés réduits ainsi obtenus, et éventuellement la salification par un acide minéral ou organique pharmaceutiquement acceptable des composés (I) ainsi obtenus.

11. Procédé selon la revendication 10, caractérisé en ce que la réduction est réalisée par le borohydrure de sodium dans l'éthanol.

12. Procédé de préparation des composés (I) et sels selon la revendication 1, 2 ou 3, caractérisé en ce qu'il comprend la réduction des composés de formule :

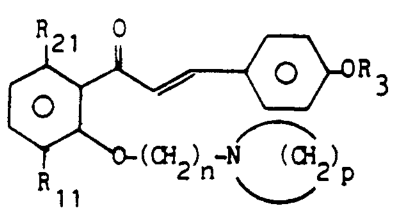

(III)

dans laquelle $R_{11}$ et $R_{21}$ ont respectivement la même signification que $R_1$ et $R_2$ dans la formule (I) définie à la revendication 1, à l'exception du groupe hydroxyle, $R_3$ = benzyle, n = 2 ou 3 et p = 4, 5 ou 6, puis l'hydrogénolyse catalytique des composés résultants et éventuellement la salification par un acide minéral ou organique pharmaceutiquement acceptable des composés (I) ainsi obtenus.

13. Procédé selon la revendication 12, caractérisé en ce que la réduction est réalisée par le borohydrure de sodium dans l' éthanol et l'hydrogénolyse catalytique est effectuée en présence de palladium sur charbon dans l'éthanol.

14. Procédé de préparation du composé (I) et sels selon la revendication 1, 2 ou 3, pour lequel $R_1$ = OH, caractérisé en ce qu'il comprend l'hydrogénolyse catalytique du composé (I) correspondant pour lequel $R_1$ = benzyloxy, et éventuellement la salification par un acide mineral ou organique pharmaceutiquement acceptable du composé (I) ainsi obtenu.

15. Procédé selon la revendication 14, caractérisé en ce que l'hydrogénolyse catalytique est effectuée en présence de palladium sur charbon, en milieu éthanolique.

16. Composés de formule :

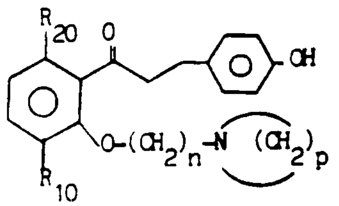

(II)

dans laquelle :

- $R_{10}$ et $R_{20}$ sont tels que :
  . soit $R_{10}$ = méthoxy, auquel cas $R_{20}$ est un groupe choisi parmi les suivants : alkyle en $C_1$-$C_4$, alkyloxy en $C_2$-$C_8$, hydroxyle, cycloalkyloxy en $C_5$-$C_7$ ;
  . soit $R_{20}$ = méthoxy, auquel cas $R_{10}$ est un groupe choisi parmi les suivants : alkyloxy en $C_2$-$C_8$, alkyloxy en $C_2$-$C_8$ substitué par un groupe méthoxy, trifluoroalkyloxy en $C_1$-$C_4$, alcényloxy en $C_3$-$C_4$, cycloalkyloxy en $C_5$-$C_7$, hydroxyle, alkylthio en $C_1$-$C_4$ ;
- n = 2 ou 3 ; et
- p = 4, 5 ou 6.

17. Composés de formule :

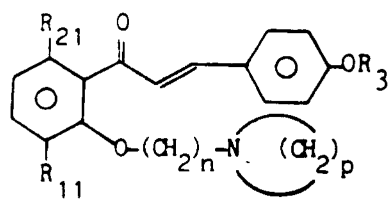

(III)

dans laquelle :

- $R_{11}$ et $R_{21}$ sont tels que :
  . soit $R_{11}$ = méthoxy, auquel cas $R_{21}$ est un groupe choisi parmi les suivants : alkyle en $C_1$-$C_4$, alkyloxy en $C_2$-$C_8$, benzyloxy, cycloalkyloxy en $C_5$-$C_7$ ;
  . soit $R_{21}$ = méthoxy, auquel cas $R_{11}$ est un groupe choisi parmi les suivants : alkyloxy en $C_2$-$C_7$, alkyloxy en $C_2$-$C_8$ substitué par un groupe méthoxy, trifluoroalkyloxy en $C_1$-$C_4$, alcényloxy en $C_3$-$C_4$, cycloalkyloxy en $C_5$-$C_7$, benzyloxy, alkylthio en $C_1$-$C_4$ ;
- n = 2 ou 3 ; et
- p = 4, 5 ou 6.

**Revendications pour les Etats contractants suivants : ES, GR**

**1.** Procédé de préparation des composés de formule générale :

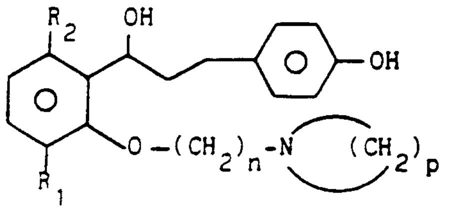

(I)

dans laquelle :

- $R_1$ et $R_2$ sont tels que :
  . soit $R_1$ représente un groupe méthoxy auquel cas $R_2$ est un groupe choisi parmi les suivants : hydroxyle ; alkyle en $C_1$-$C_4$ ; alkyloxy en $C_2$-$C_8$ ; cycloalkyloxy en $C_5$-$C_7$ ; benzyloxy ;
  . soit $R_2$ représente un groupe méthoxy auquel cas $R_1$ représente un groupe choisi parmi les suivants : hydroxyle ; alkyloxy en $C_2$-$C_8$ ; alkyloxy en $C_2$-$C_8$ substitué par un groupe méthoxy ; trifluoroalkyloxy en $C_1$-$C_4$ ; alcényloxy en $C_3$-$C_4$ ; cycloalkyloxy en $C_5$-$C_7$ ; benzyloxy ; alkylthio en $C_1$-$C_4$ ;
- n = 2 ou 3 ; et
- p = 4, 5 ou 6,

ainsi que de leurs sels d' addition avec les acides minéraux ou organiques pharmaceutiquement acceptables, caractérisé en ce qu'il comprend l'une des réactions (a)-(e) suivantes :

(a) réduction des composés de formule :

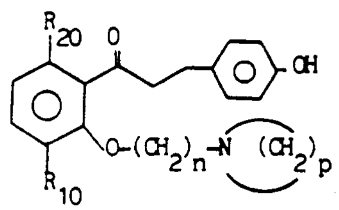

(II)

dans laquelle $R_{10}$ et $R_{20}$ ont respectivement la même signification que $R_1$ et $R_2$ dans la formule (I) définie ci-dessus, à l'exception du groupe benzyloxy, n = 2 ou 3 et p = 4, 5 ou 6,

(b) réduction des composés de formule :

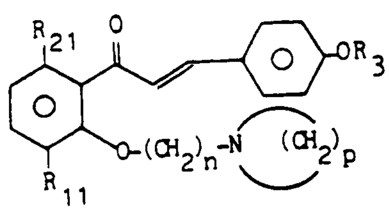

(III)

dans laquelle $R_{11}$ et $R_{21}$ ont respectivement la même signification que $R_1$ et $R_2$ dans la formule (I) définie ci-dessus, à l'exception du groupe hydroxyle, $R_3$ = H, n = 2 ou 3 et p = 4, 5 ou 6,

(c) réduction des composés de formule :

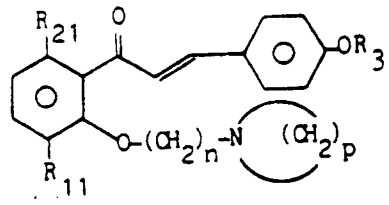

(III)

dans laquelle $R_{11}$ et $R_{21}$ ont respectivement la même signification que $R_1$ et $R_2$ dans la formule (I) définie ci-dessus, à l'exception du groupe hydroxyle, $R_3$ = tétrahydropyranyl-2, n = 2 et p = 4, 5 ou 6, puis hydrolyse acide des composés ainsi obtenus,

(d) réduction des composés de formule :

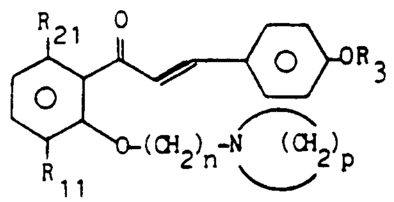

(III)

dans laquelle $R_{11}$ et $R_{21}$ ont respectivement la même signification que $R_1$ et $R_2$ dans la formule (I) définie ci-dessus, à l'exception du groupe hydroxyle, $R_3$ = benzyle, n = 2 ou 3 et p = 4, 5 ou 6, puis hydrogénolyse catalytique des composés résultants,

(e) hydrogénolyse catalytique du composé de formule (I) ci-dessus pour lequel $R_1$ = benzyloxy, $R_2$ = méthoxy, n = 2 ou 3 et p = 4, 5 ou 6, et éventuellement la salification par un acide mineral ou organique pharmaceutiquement acceptable des composés (I) obtenus conformément à (a), (b), (c), (d) et (e).

**2.** Procédé selon la revendication 1, caractérisé en ce que la réduction (a) est réalisée par le borohydrure de sodium en milieu alcoolique, en présence de NaOH.

**3.** Procédé selon la revendication 1, caractérisé en ce que la réduction (b) est réalisée par le borohydrure de sodium, en milieu éthanolique et en présence de pyridine et de NaOH.

**4.** Procédé selon la revendication 1, caractérisé en ce que la réduction (c) est réalisée par le borohydrure de sodium dans l'éthanol.

**5.** Procédé selon la revendication 1, caractérisé en ce que la réduction sous (d) est réalisée par le borohydrure de sodium dans l'éthanol et l'hydrogénolyse catalytique sous (d) est effectuée en présence de palladium sur charbon dans l'éthanol.

**6.** Procédé selon la revendication 1, caractérisé en ce que l'hydrogénolyse catalytique (e) est effectuée en présence de palladium sur charbon, en milieu éthanolique.

**7.** Procédé selon l'une des revendications 1 à 6, pour la préparation des composés de formule (I) dans laquelle $R_1$ = méthoxy et $R_2$ est un groupe choisi parmi les suivants : benzyloxy, hydroxyle, n-butyloxy, n-propyloxy, i-propyloxy, n-octyloxy, cyclohexyloxy, éthoxy, méthyle, éthyle.

**8.** Procédé selon l'une des revendications 1 à 6, pour la préparation des composés de formule (I) dans laquelle $R_2$ = méthoxy et $R_1$ est un groupe choisi parmi les suivants : trifluoro-2,2,2 éthoxy, éthoxy, n-propyloxy, allyloxy, benzyloxy, hydroxyle, méthoxy-2 éthoxy, cyclohexyloxy, méthylthio, éthylthio.

**9.** Procédé de préparation d'une composition pharmaceutique, caractérisé en ce qu'il consiste à combiner au moins un composé ou sel obtenu conformément au procédé selon l'une des revendications 1 à 6,

avec un excipient physiologiquement acceptable.

**Claims**
**Claims for the following Contracting States : AT, BE, CH, DE, FR, GB, IT, LI, LU, NL, SE**

1. Compounds of general formula:

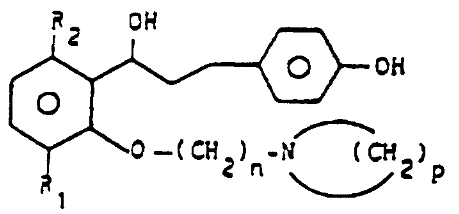

(I)

in which:
- R1 and R2 are such that:
  . either R1 represents a methoxy group in which case R2 is a group selected from among the following: hydroxyl; C1-C4 alkyl; C2-C8 alkyloxy; C5-C7 cycloalkyloxy; benzyloxy;
  . or R2 represents a methoxy group in which case R1 represents a group selected from among the following: hydroxyl; C2-C8 alkyloxy; C2-C8 alkyloxy substituted by a methoxy group; C1-C4 trifluoroalkyloxy; C3-C4 alkenyloxy; C5-C7 cycloalkyloxy; benzyloxy; C1-C4 alkylthio;
- n = 2 or 3; and
- p = 4, 5 or 6,

together with their added salts with the pharmaceutically acceptable mineral or organic acids.

2. Compounds and salts according to Claim 1, for which R1 = methoxy and R2 is a group selected from among the following: benzyloxy, hydroxyl, n-butyloxy, n-propyloxy, i-propyloxy, n-octyloxy, cyclohexyloxy, ethoxy, methyl, ethyl.

3. Compounds and salts according to Claim 1, for which R2 = methoxy and R1 is a group selected from among the following: trifluoro-2,2,2 ethoxy, ethoxy, n-propyloxy, allyloxy, benzyloxy, hydroxyl, methoxy-2 ethoxy, cyclohexyloxy, methylthio, ethylthio.

4. The use of the pharmaceutically acceptable compounds and salts according to any one of Claims 1, 2 or 3, for the preparation of a medicine.

5. A pharmaceutical compound, characterised in that it comprises, by way of active principle, at least one compound or salt according to any one of Claims 1, 2 or 3 and a physiologically acceptable excipient.

6. A method of preparing compounds (I) and salts according to any one of Claims 1, 2 or 3, characterised in that it comprises the reduction of the compounds of the formula:

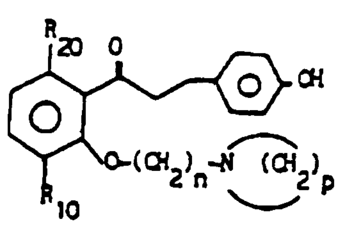

(II)

in which R10 and R20 respectively have the same significance as R1 and R2 in the formula (I) defined in Claim 1, with the exception of the benzyloxy group, n = 2 or 3 and p = 4, 5 or 6, this reduction possibly being followed by salification by a pharmaceutically acceptable mineral or organic acid of the compounds (I) thus obtained.

7. A method according to Claim 6, characterised in that the reduction is effected by sodium borohydride in an alcohol environment, in the presence of NaOH.

8. A method for the preparation of the compounds (I) and salts according to any one of Claims 1, 2 or 3, characterised in that it comprises the reduction of the compounds of the formula:

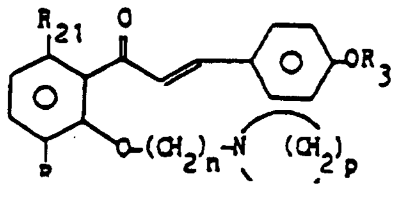

(III)

in which R11 and R21 respectively have the same significance as R1 and R2 in the formula (I) defined, in Claim 1, with the exception of the hydroxyl group, R3 = H, n = 2 or 3 and p = 4, 5 or 6, this reduction possibly being followed by the salification by a pharmaceutically acceptable mineral or organic acid of the compounds (I) thus obtained.

9. A method according to Claim 8, characterised in that the reduction is effected by sodium borohydride in an ethanol environment and in the presence of pyridine and NaOH.

10. A method for the preparation of the compounds (I) and salts according to any one of Claims 1, 2 or 3, characterised in that it comprises the reduction of the compounds of the formula:

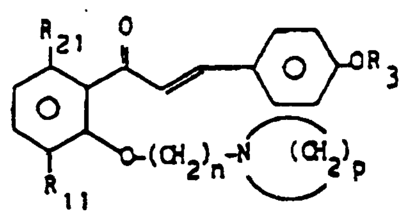

(III)

in which R11 and R21 respectively have the same significance as R1 and R2 in the formula (I) defined in Claim 1, with the exception of the hydroxyl group, R3 = tetrahydropyranyl-2, n = 2 or 3 and p = 4, 5 or 6, then the acid hydrolysis of the reduced compounds thus obtained and possibly the salification by a pharmaceutically acceptable mineral or organic acid of the compounds (I) thus obtained.

11. A method according to Claim 10, characterised in that the reduction is effected by sodium borohydride in ethanol.

12. A method for the preparation of the compounds (I) and salts according to any one of Claims 1, 2 or 3, characterised in that it comprises the reduction of the compounds of the formula:

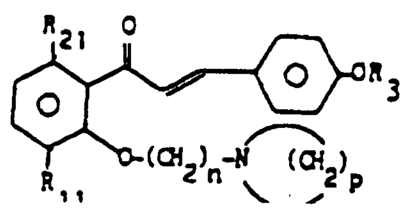

(III)

in which R11 and R21 respectively have the same significance as R1 and R2 in the formula (I) defined in Claim 1, with the exception of the hydroxyl group, R3 = benzyl, n = 2 or 3 and p - 4, 5 or 6, then the catalytic hydrogenation of the resulting compounds and possibly salification by a pharmaceutically acceptable mineral or organic acid of the compounds (I) thus obtained.

**13.** A method according to Claim 12, characterised in that the reduction is effected by sodium borohydride in ethanol and catalytic hydrogenation is effected in the presence of palladium on carbon in ethanol.

**14.** A method of preparation of the compound (I) and salts according to any one of the Claims 1, 2 and 3, for which R1 = OH, characterised in that it comprises the catalytic hydrogenation of the corresponding compound (I) for which R1 = benzyloxy and possibly salification by a pharmaceutically acceptable mineral or organic acid of the compound (I) thus obtained.

**15.** A method according to Claim 14, characterised in that the catalytic hydrogenation is effected in the presence of palladium on carbon in an ethanol environment.

**16.** Compounds of the formula:

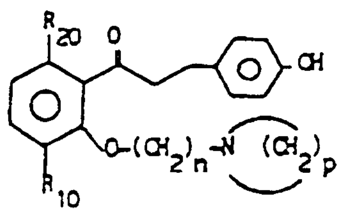

(II)

in which:

- R10 and R20 are such that:
  . either R10 = methoxy, in which case R20 is a group selected from among the following : C1-C4 alkyl; C2-C8 alkyloxy, hydroxyl, C5-C7 cycloalkyloxy;
  . or R20 = methoxy, in which case R10 is a group selected from among the following: C2-C8 alkyloxy, C2-C8 alkyloxy substituted by a methoxy group, C1-C4 trifluoroalkyloxy, C3-C4 alkenyloxy, C5-C7 cycloalkyloxy, hydroxyl, C1-C4 alkylthio;
- n = 2 or 3; and
- p = 4, 5 or 6.

**17.** Compounds of the formula:

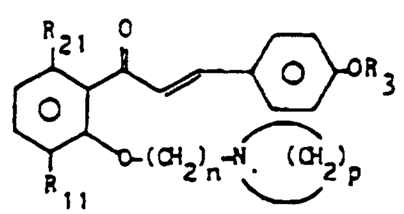

(III)

in which:

- R11 and R21 are such that:
  . either R11 = methoxy in which case R21 is a group selected from among the following: C1-C4 alkyl, C2-C8 alkyloxy, benzyloxy, C5-C7 cycloalkyloxy;
  . or R21 = methoxy, in which case R11 is a group selected from among the following: C2-C7 alkyloxy, C2-C8 alkyloxy substituted by a methoxy group, C1-C4 trifluoroalkyloxy, C3-C4 alkenyloxy, C5-C7 cycloalkyloxy, benzyloxy, C1-C4 alkylthio;
- n = 2 or 3; and
  p = 4, 5 or 6

**Claims for the following Contracting States : ES, GR**

**1.** A method of preparation of compounds of the general formula:

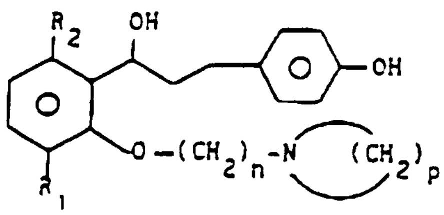

(I)

in which:

- R1 and R2 are such that:
  - . either R1 represents a methoxy group in which case R2 is a group selected from among the following: hydroxyl; C1-C4 alkyl; C2-C8 alkyloxy; C5-C7 cycloalkyloxy; benzyloxy;
  - . or R2 represents a methoxy group in which case R1 represents a group selected from among the following: hydroxyl; C2-C8 alkyloxy; C2-C8 alkyloxy substituted by a methoxy group; C1-C4 trifluoroalkyloxy; C3-C4 alkenyloxy; C5-C7 cycloalkyloxy; benzyloxy; C1-C4 akylthio;
- n = 2 or 3; and
- p = 4, 5 or 6,

together with their added salts with the pharmaceutically acceptable mineral or organic acid, characterised in that it comprises one of the following reactions (a)-(e):

(a) the reduction of the compounds of the formula:

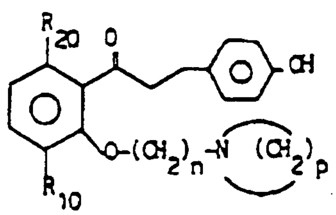

(II)

in which R10 and R20 respectively have the same significance as R1 and R2 in the formula (I) defined above, with the exception of the benzyloxy group, n = 2 or 3 and p = 4, 5 or 6,

(b) the reduction of the compounds of the formula:

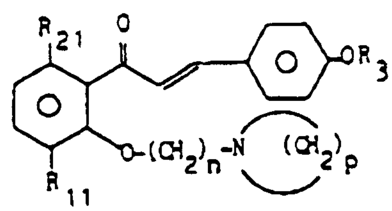

(III)

in which R11 and R21 respectively have the same significance as R1 and R2 in the formula (I) defined above, with the exception of the hydroxyl group, R3 = H, n = 2 or 3 and p = 4, 5 or 6,

(c) the reduction of the compounds of the formula:

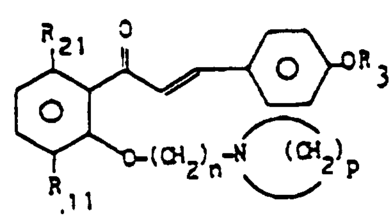

(III)

in which R11 and R21 respectively have the same significance as R1 and R2 in the formula (I) defined above, with the exception of the hydroxyl group, R3 = tetrahydropyranyl-2, n = 2 and p = 4, 5 or 6, then the acid hydrolysis of the compounds thus obtained,

(d) the reduction of the compounds of the formula:

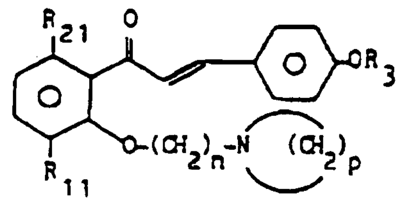

(III)

in which R11 and R21 respectively have the same significance as R1 and R2 in the formula (I) defined above, with the exception of the hydroxyl group, R3 = benzyl, n = 2 or 3 and p = 4, 5 or 6, then the catalytic hydrogenation of the resulting compounds.

(e) the catalytic hydrogenation of the compound of the formula (I) above for which R1 = benzyloxy, R2 = methoxy, n = 2 or 3 and p = 4, 5, or 6, and possibly the salification by a pharmaceutically acceptable mineral or organic acid of the compounds (I) obtained in accordance with (a), (b), (c), (d) and (e).

2. A method according to Claim 1, characterised in that the reduction in (a) is effected by sodium borohydride in an alcohol environment, in the presence of NaOH.

3. A method according to Claim 1, characterised in that the reduction in (b) is effected by sodium borohydride in an ethanol environment and in the presence of pyridine and NaOH.

4. A method according to Claim 1, characterised in that the reduction in (c) is effected by sodium borohydride in ethanol.

5. A method according to Claim 1, characterised in that the reduction (d)is effected by sodium borohydride in ethanol and the catalytic hydrogenation in (d) is effected in the presence of palladium on carbon in ethanol.

6. A method according to Claim 1, characterised in that the catalytic hydrogenation (e) is effected in the presence of palladium on carbon in an ethanol environment.

7. A method according to any one of Claims 1 to 6, for the preparation of the compounds of the formula (I) in which R1 = methoxy and R2 is a group selected from among the following: benzyloxy, hydroxyl, n-butyloxy, n-propyloxy, i-propyloxy, n-octyloxy, cyclohexyloxy, ethoxy, methyl, ethyl.

8. A method according to any one of Claims 1 to 6 for the preparation of the compounds of the formula (I) in which R2 = methoxy and R1 is a group selected from among the following: trifluoro-2,2,2 ethoxy, ethoxy, n-propyloxy, allyloxy, benzyloxy, hydroxyl, methoxy-2 ethoxy, cyclohexyloxy, methylthio, ethylthio.

9. A method for the preparation of a pharmaceutical composition, characterised in that it consists in combining at least one compound or salt obtained in accordance with the method according to any one of the Claims 1 to 6, with a physiologically acceptable excipient.

**Patentansprüche**

**Patentansprüche für folgende Vertragsstaaten : AT, BE, CH, DE, FR, GB, IT, LI, LU, NL, SE**

1. Verbindungen der allgemeinen Formel:

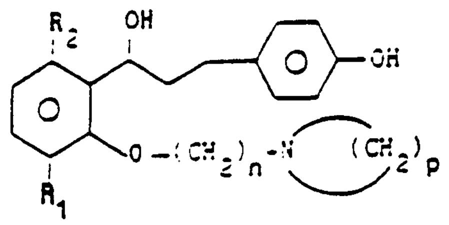

(I)

in welcher:
$R_1$ und $R_2$ folgendermaßen sind:
entweder $R_1$ bedeutet eine Methoxygruppe, in welchem Fall $R_2$ eine Gruppe ist, ausgewählt unter den folgenden: Hydroxyl; $C_1$-$C_4$-Alkyl; $C_2$-$C_8$-Alkyloxy; $C_5$-$C_7$-Cycloalkyloxy; Benzyloxy;
oder $R_2$ bedeutet eine Methoxygruppe, in welchem Fall $R_1$ eine Gruppe bedeutet, ausgewählt unter den folgenden: Hydroxyl; $C_2$-$C_8$-Alkyloxy; $C_2$-$C_8$-Alkyloxy, substituiert durch eine Methoxygruppe; $C_1$-$C_4$-Trifluoralkyloxy; $C_3$-$C_4$-Alkenyloxy; $C_5$-$C_7$-Cycloalkyloxy; Benzyloxy; $C_1$-$C_4$-Alkylthio;
$n = 2$ oder 3; und
$p = 4$, 5 oder 6,
sowie ihre Additionssalze mit pharmazeutisch annehmbaren anorganischen oder organischen Säuren.

2. Verbindungen und Salze nach Anspruch 1, für die $R_1$ = Methoxy und $R_2$ eine Gruppe ist, ausgewählt unter den folgenden: Benzyloxy, Hydroxyl, n-Butyloxy, n-Propyloxy, i-Propyloxy, n-Octyloxy, Cyclohexyloxy, Ethoxy, Methyl, Ethyl.

3. Verbindungen und Salze nach Anspruch 1, für die $R_2$ = Methoxy und $R_1$ eine Gruppe ist, ausgewählt unter den folgenden: 2,2,2-Trifluorethoxy, Ethoxy, n-Propyloxy, Allyloxy, Benzyloxy, Hydroxyl, 2-Methoxy-ethoxy, Cyclohexyloxy, Methylthio, Ethylthio.

4. Verwendung der Verbindungen und pharmazeutisch annehmbaren Salze nach Anspruch 1, 2 oder 3, zur Herstellung eines Arzneimittels.

5. Pharmazeutische Zusammensetzung, **dadurch gekennzeichnet,** daß sie als Wirkstoff mindestens eine Verbindung oder Salz nach Anspruch 1, 2 oder 3 und ein physiologisch annehmbares Excipiens umfaßt.

6. Verfahren zur Herstellung von Verbindungen (I) und Salzen nach Anspruch 1, 2 oder 3, **dadurch gekennzeichnet,** daß es die Reduktion von Verbindungen der Formel:

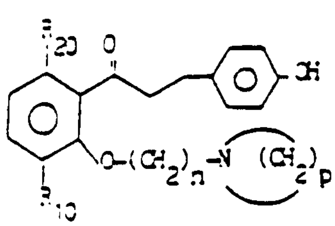

(II)

in welcher $R_{10}$ und $R_{20}$ jeweils die gleiche Bedeutung wie $R_1$ und $R_2$ in Formel (I), welche in Anspruch 1 definiert ist, haben, mit Ausnahme der Benzyloxygruppe, $n = 2$ oder 3 und $p = 4$, 5 oder 6, umfaßt, wobei auf diese Reduktion eventuell eine Bildung von Salzen der so erhaltenen Verbindungen (I) durch eine pharmazeutisch annehmbare anorganische oder organische Säure folgt.

7. Verfahren nach Anspruch 6, **dadurch gekennzeichnet,** daß die Reduktion durch Natriumborhydrid in alkoholischer Umgebung in Gegenwart von NaOH durchgeführt wird.

8. Verfahren zur Herstellung von Verbindungen (I) und Salzen nach Anspruch 1, 2 oder 3, **dadurch gekennzeichnet,** daß es die Reduktion von Verbindungen der Formel:

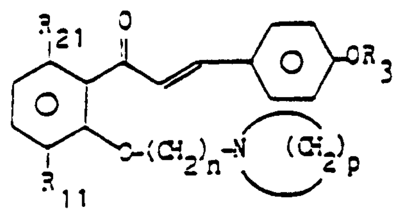

(III)

in welcher $R_{11}$ und $R_{21}$ jeweils die gleiche Bedeutung wie $R_1$ und $R_2$ in Formel (I), die in Anspruch 1 definiert ist, haben, mit Ausnahme der Hydroxylgruppe, $R_3$ = H, n = 2 oder 3 und p = 4, 5 oder 6, umfaßt, wobei auf diese Reduktion eventuell eine Bildung von Salzen der so erhaltenen Verbindungen (I) durch eine pharmazeutisch annehmbare anorganische oder organische Säure folgt.

9. Verfahren nach Anspruch 8, **dadurch gekennzeichnet,** daß die Reduktion durch Natriumborhydrid in ethanolischer Umgebung und in Gegenwart von Pyridin und NaOH durchgeführt wird.

10. Verfahren zur Herstellung von Verbindungen (I) und Salzen nach Anspruch 1, 2 oder 3, **dadurch gekennzeichnet,** daß es die Reduktion von Verbindungen der Formel:

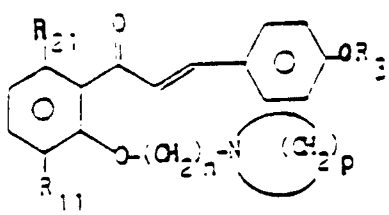

(III)

in welcher $R_{11}$ und $R_{21}$ jeweils die gleiche Bedeutung wie $R_1$ und $R_2$ in Formel (I), die in Anspruch 1 definiert ist, haben, mit Ausnahme der Hydroxylgruppe, $R_3$ = Tetrahydropyranyl-2, n = 2 oder 3 und p = 4, 5 oder 6, dann die saure Hydrolyse der so erhaltenen reduzierten Verbindungen und eventuell die Bildung von Salzen der so erhaltenen Verbindungen (I) durch eine pharmazeutisch annehmbare anorganische oder organische Säure umfaßt.

11. Verfahren nach Anspruch 10, **dadurch gekennzeichnet,** daß die Reduktion durch Natriumborhydrid in Ethanol durchgeführt wird.

12. Verfahren zur Herstellung von Verbindungen (I) und Salzen nach Anspruch 1, 2 oder 3, **dadurch gekennzeichnet,** daß es die Reduktion von Verbindungen der Formel:

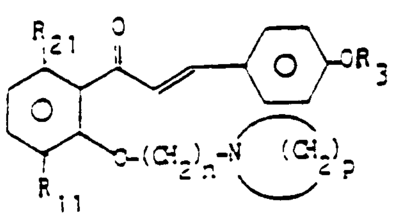

(III)

in welcher $R_{11}$ und $R_{21}$ jeweils die gleiche Bedeutung wie $R_1$ und $R_2$ in Formel (I), die in Anspruch 1 definiert ist, haben, mit Ausnahme der Hydroxylgruppe, $R_3$ = Benzyl, n = 2 oder 3 und p = 4, 5 oder

6, dann die katalytische Hydrogenolyse der daraus hervorgehenden Verbindungen und eventuell eine Bildung von Salzen der so erhaltenen Verbindungen (I) durch eine pharmazeutisch annehmbare anorganische oder organische Säure umfaßt.

**13.** Verfahren nach Anspruch 12, **dadurch gekennzeichnet,** daß die Reduktion durch Natriumborhydrid in Ethanol durchgeführt wird und die katalytische Hydrogenolyse in Gegenwart von Palladium auf Kohlenstoff in Ethanol ausgeführt wird.

**14.** Verfahren zur Herstellung der Verbindung (I) und Salzen nach Anspruch 1, 2 oder 3, für welche $R_1$ = OH, **dadurch gekennzeichnet,** daß es die katalytische Hydrogenolyse der entsprechenden Verbindung (I), für die $R_1$ = Benzyloxy, und eventuell die Bildung von Salzen der so erhaltenen Verbindung (I) durch eine pharmazeutisch annehmbare anorganische oder organische Säure umfaßt.

**15.** Verfahren nach Anspruch 14, **dadurch gekennzeichnet,** daß die katalytische Hydrogenolyse in Gegenwart von Palladium auf Kohlenstoff in ethanolischer Umgebung ausgeführt wird.

**16.** Verbindungen der Formel:

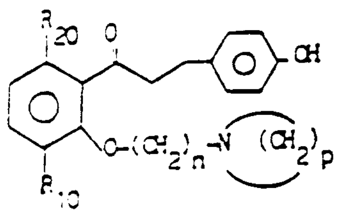

(II)

in welcher:
$R_{10}$ und $R_{20}$ folgendermaßen sind:
entweder $R_{10}$ = Methoxy, in welchem Fall $R_{20}$ eine Gruppe ist, ausgewählt unter den folgenden: $C_1$-$C_4$-Alkyl, $C_2$-$C_8$-Alkyloxy, Hydroxyl, $C_5$-$C_7$-cycloalkyloxy;
oder $R_{20}$ = Methoxy, in welchem Fall $R_{10}$ eine Gruppe ist, ausgewählt unter den folgenden: $C_2$-$C_8$-Alkyloxy, $C_2$-$C_8$-Alkyloxy, das durch eine Methoxygruppe substituiert ist, $C_1$-$C_4$-Trifluoralkyloxy, $C_3$-$C_4$-Alkenyloxy, $C_5$-$C_7$-Cycloalkyloxy, Hydroxyl, $C_1$-$C_4$-Alkylthio;
n = 2 oder 3; und
p = 4, 5 oder 6.

**17.** Verbindungen der Formel:

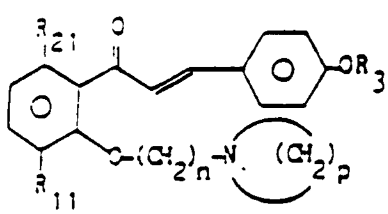

(III)

in welcher:
$R_{11}$ und $R_{21}$ folgendermaßen sind:
Entweder $R_{11}$ = Methoxy, in welchem Fall $R_{21}$ eine Gruppe ist, ausgewählt unter den folgenden: $C_1$-$C_4$-Alkyl, $C_2$-$C_8$-Alkyloxy, Benzyloxy, $C_5$-$C_7$-Cycloalkyloxy;
oder $R_{21}$ = Methoxy, in welchem Fall $R_{11}$ eine Gruppe ist, ausgewählt unter den folgenden: $C_2$-$C_7$-Alkyloxy, $C_2$-$C_8$-Alkyloxy, das durch eine Methoxygruppe substituiert ist, $C_1$-$C_4$-Trifluoralkyloxy, $C_3$-$C_4$-Alkenyloxy, $C_5$-$C_7$-Cycloalkyloxy, Benzyloxy, $C_1$-$C_4$-Alkylthio;
n = 2 oder 3; und
p = 4, 5 oder 6.

**Patentansprüche für folgende Vertragsstaaten : ES, GR**

1. Verfahren zur Herstellung von Verbindungen der allgemeinen Formel:

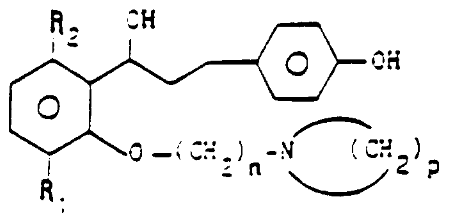

(I)

in welcher:
$R_1$ und $R_2$ folgendermaßen sind:
entweder $R_1$ bedeutet eine Methoxygruppe, in welchem Fall $R_2$ eine Gruppe ist, ausgewählt unter den folgenden: Hydroxyl; $C_1$-$C_4$-Alkyl; $C_2$-$C_8$-Alkyloxy; $C_5$-$C_7$- Cycloalkyloxy; Benzyloxy;
oder $R_2$ bedeutet eine Methoxygruppe, in welchem Fall $R_1$ eine Gruppe bedeutet, ausgewählt unter den folgenden: Hydroxyl; $C_2$-$C_8$-Alkyloxy; $C_2$-$C_8$-Alkyloxy, substituiert durch eine Methoxygruppe; $C_1$-$C_4$-Trifluoralkyloxy; $C_3$-$C_4$-Alkenyloxy; $C_5$-$C_7$-Cycloalkyloxy; Benzyloxy; $C_1$-$C_4$-Alkylthio;
$n = 2$ oder 3; und
$p = 4$, 5 oder 6;
sowie ihren Additionssalzen mit den pharmazeutisch annehmbaren anorganischen oder organischen Säuren, **dadurch gekennzeichnet,** daß es eine der folgenden Reaktionen (a)-(e) umfaßt:
(a) Reduktion von Verbindungen der Formel:

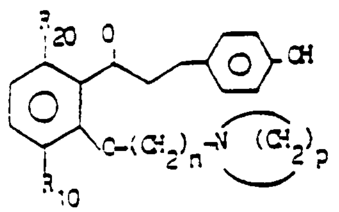

(II)

in welcher $R_{10}$ und $R_{20}$ jeweils die gleiche Bedeutung wie $R_1$ und $R_2$ in der Formel (I), die oben definiert ist, haben, mit Ausnahme der Benzyloxygruppe, $n = 2$ oder 3 und $p = 4$, 5 oder 6,
(b) Reduktion von Verbindungen der Formel:

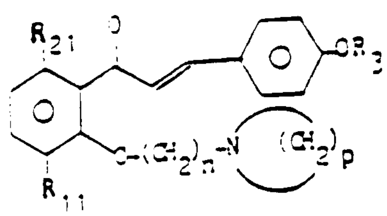

(III)

in welcher $R_{11}$ und $R_{21}$ jeweils die gleiche Bedeutung wie $R_1$ und $R_2$ in der Formel (I), die oben definiert ist, haben, mit Ausnahme der Hydroxylgruppe, $R_3$ = H, $n = 2$ oder 3 und $p = 4$, 5 oder 6,

(c) Reduktion von Verbindungen der Formel:

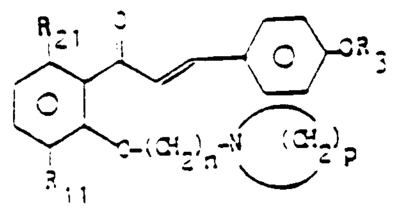

(III)

in welcher $R_{11}$ und $R_{21}$ jeweils die gleiche Bedeutung wie $R_1$ und $R_2$ in der Formel (I), die oben definiert ist, haben, mit Ausnahme der Hydroxylgruppe, $R_3$ = Tetrahydropyranyl-2, n = 2 und p = 4, 5 oder 6, dann saure Hydrolyse der so erhaltenen Verbindungen,

(d) Reduktion von Verbindungen der Formel:

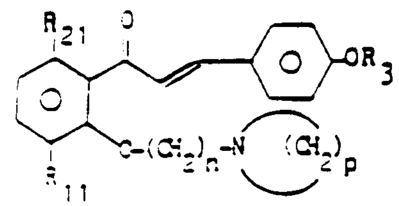

(III)

in welcher $R_{11}$ und $R_{21}$ jeweils die gleiche Bedeutung wie $R_1$ und $R_2$ in der Formel (I), die oben definiert ist, haben, mit Ausnahme der Hydroxylgruppe, $R_3$ = Benzyl, n = 2 oder 3 und p = 4, 5 oder 6, dann katalytische Hydrogenolyse der daraus hervorgehenden Verbindungen

(e) katalytische Hydrogenolyse der Verbindung der Formel (I) oben, für welche $R_1$ = Benzyloxy, $R_2$ = Methoxy, n = 2 oder 3 und p = 4, 5 oder 6, und eventuell die Bildung von Salzen der Verbindungen (I), die gemäß (a), (b), (c), (d) und (e) erhalten wurden, durch eine pharmazeutisch annehmbare anorganische oder organische Säure.

**2.** Verfahren nach Anspruch 1, **dadurch gekennzeichnet,** daß die Reduktion (a) durch Natriumborhydrid in alkoholischer Umgebung in Gegenwart von NaOH durchgeführt wird.

**3.** Verfahren nach Anspruch 1, **dadurch gekennzeichnet,** daß die Reduktion (b) durch Natriumborhydrid in ethanolischer Umgebung und in Gegenwart von Pyridin und NaOH durchgeführt wird.

**4.** Verfahren nach Anspruch 1, **dadurch gekennzeichnet,** daß die Reduktion (c) durch Natriumborhydrid in Ethanol durchgeführt wird.

**5.** Verfahren nach Anspruch 1, **dadurch gekennzeichnet,** daß die Reduktion (d) durch Natriumborhydrid in Ethanol durchgeführt wird und die katalytische Hydrogenolyse (d) in Gegenwart von Palladium auf Kohlenstoff in Ethanol ausgeführt wird.

**6.** Verfahren nach Anspruch 1, **dadurch gekennzeichnet,** daß die katalytische Hydrogenolyse (e) in Gegenwart von Palladium auf Kohlenstoff in ethanolischer Umgebung ausgeführt wird.

**7.** Verfahren nach einem der Ansprüche 1 bis 6, zur Herstellung von Verbindungen der Formel (I), in welcher $R_1$ = Methoxy und $R_2$ eine Gruppe ist, ausgewählt unter den folgenden: Benzyloxy, Hydroxyl, n-Butyloxy, n-Propyloxy, i-Propyloxy, n-Octyloxy, Cyclohexyloxy, Ethoxy, Methyl, Ethyl.

**8.** Verfahren nach einem der Ansprüche 1 bis 6, zur Herstellung von Verbindungen der Formel (I) in welcher $R_2$ = Methoxy und $R_1$ eine Gruppe ist, ausgewählt unter den folgenden: 2,2,2-Trifluorethoxy, Ethoxy, n-Propyloxy, Allyloxy, Benzyloxy, Hydroxyl, 2-Methoxy-ethoxy, Cyclohexyloxy, Methylthio, Ethylthio.

9. Verfahren zur Herstellung einer pharmazeutischen Zusammensetzung, **dadurch gekennzeichnet,** daß es darin besteht, mindestens eine Verbindung oder Salz, welche gemäß dem Verfahren nach einem der Ansprüche 1 bis 6 erhalten wurden, mit einem physiologisch annehmbaren Excipiens zu kombinieren.